(19)

Europäisches Patentamt
European Patent Office
Office européen des brevets

(11) **EP 4 740 987 A2**

(12) **EUROPEAN PATENT APPLICATION**

(43) Date of publication:
**13.05.2026 Bulletin 2026/20**

(51) International Patent Classification (IPC):
***A61M 16/10*** *(2006.01)*

(21) Application number: 25224682.2

(22) Date of filing: **18.02.2016**

(52) Cooperative Patent Classification (CPC):
**A61M 16/104; A61M 16/0051; A61M 16/026;
A61M 16/0672; A61M 16/16;** A61M 2016/0027;
A61M 2016/003; A61M 2202/0208;
A61M 2205/3331; A61M 2205/502; A61M 2205/581;
A61M 2205/583

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.02.2015 US 201562117817 P
31.03.2015 US 201562140592 P
31.03.2015 US 201562140598 P**

(62) Document number(s) of the earlier application(s) in
accordance with Art. 76 EPC:
**16752716.7 / 3 242 703**

(71) Applicant: **Fisher & Paykel Healthcare Limited
2013 Auckland (NZ)**

(72) Inventors:
• **PAYTON, Matthew Jon
2013 Auckland (NZ)**
• **EVANS, Alicia Jerram Hunter
2013 Auckland (NZ)**
• **BARNES, Thomas, Henrich
2013 Auckland (NZ)**
• **CHEUNG, Dexter Chi Lun
2013 Auckland (NZ)**
• **WHITE, Craig Karl
2013 Auckland (NZ)**
• **WILLIAMS, Anthony Brendan
2013 Auckland (NZ)**

• **GULLIVER, Laurence
2013 Auckland (NZ)**
• **BARRACLOUGH, Michael
2013 Auckland (NZ)**
• **CHURCH, Jonathan Mark
2013 Auckland (NZ)**
• **HARWOOD, Jonathan David
2013 Auckland (NZ)**
• **OLDFIELD, Samantha Dale
2013 Auckland (NZ)**
• **SPENCE, Callum James Thomas
2013 Auckland (NZ)**
• **ASSI, Milanjot Singh
2013 Auckland (NZ)**

(74) Representative: **Forresters IP LLP
Skygarden
Erika-Mann-Straße 11
80636 München (DE)**

Remarks:
•This application was filed on 17-12-2025 as a
divisional application to the application mentioned
under INID code 62.
•Claims filed after the date of filing of the application /
after the date of receipt of the divisional application
(Rule 68(4) EPC)

(54) **FLOW THERAPY SYSTEM**

(57)    There is disclosed system for oxygenating a patient in relation to anaesthesia using high flow gas delivery. The system has a flow source, and a controller for determining oxygenation requirements of the patient before or during anaesthesia. A method of oxygenating a patient in relation to anaesthesia using high flow gas delivery is also disclosed. The method determines oxygenation requirements of the patient before or during anaesthesia.

**EP 4 740 987 A2**

**Description**

**TECHNICAL FIELD**

The present disclosure generally relates to respiratory gas therapy. More particularly, the present disclosure relates to respiratory gas therapy systems, apparatuses, kits and methods for treating patients undergoing anaesthetic or anaesthesia related procedures.

**DESCRIPTION OF THE RELATED ART**

Intubation is often practiced on patients who are unable to spontaneously breathe. The inability to breathe may be the result of one or several factors, including severe illness, injury, or deep sedation through the use of a general anaesthetic agent. A challenge for anaesthesiologists, including emergency, intensive care and surgical physicians, is to secure a tracheal tube (i.e., intubate) rapidly without causing hypoxia. In the best case intubation may take 45-60 seconds; however, in other cases, particularly if the patient's airway is difficult to traverse (for example, due to cancer, obesity, or severe injury), intubation can take much longer. Patients who are breathing room air will desaturate within this time between sedative/paralytic administration and achieving a secured airway. A patient desaturates when the oxygen concentration in their blood reduces.

To prevent hypoxemia during intubation, a medical professional performing the intubation will often pre-oxygenate the patient to be intubated by applying a face mask and delivering oxygen for a period of time until the patient's blood oxygen saturation level (measured using, for example, near infrared spectroscopy, pulse oximetry, or any other suitable process and equipment) reaches approximately 100%. Pre-oxygenation can provide a buffer against undesirable declines in oxygen saturation, but for long intubation procedures, it is often necessary to interrupt the intubation process and reapply the face mask to again increase the patient's oxygen saturation level to adequate levels. The interruption of the intubation process, which can happen several times for a difficult intubation process, can be frustrating to the medical professional. Additionally, the patient can experience rises in blood carbon dioxide due to the poor management of physiological dead space. Similar difficulties can be encountered with sedated spontaneously breathing patients undergoing, for example, upper endoscopies.

Pre-oxygenation with 100% oxygen or almost 100%, (for example 90% or higher) has been shown to markedly increase the duration of safe apnea - defined as the time until a patient reaches a blood oxygen saturation level of 88-90%. Saturations below this level can rapidly deteriorate to critical levels (<70%) on the oxyhemoglobin dissociation curve posing significant risk to the patient. Ideally patients should continue to receive pre-oxygenation until they achieve greater than 90% end-tidal oxygen levels, showing the lungs have been denitrogenised and an oxygen reservoir established in the functional residual capacity of the lungs. Denitrogenation of the lungs creates an alveolar oxygen reservoir that serves to maintain oxygen saturation levels for a small post-ventilatory window.

Patients may lose respiratory function during anaesthesia, or sedation, or more generally during certain medical procedures. Prior to a medical procedure a patient may be pre-oxygenated by a medical professional to provide a reservoir of oxygen saturation, and this pre-oxygenation is generally carried out with a bag and a face mask. Once under general anaesthesia, patients must be intubated to ventilate the patient. In some cases, intubation is completed in 30 to 60 seconds, but in other cases, particularly if the patient's airway is difficult to traverse (for example, due to cancer, severe injury, obesity or spasm of the neck muscles), intubation will take significantly longer. While pre-oxygenation provides a buffer against declines in oxygen saturation, for long intubation procedures, it is necessary to interrupt the intubation process and reapply the face mask to increase the patient's oxygen saturation to adequate levels. The interruption of the intubation process may happen several times for difficult intubation processes, which is time consuming and puts the patient at severe health risk. After approximately three attempts at intubation the medical procedure will be abandoned.

Patients require oxygenation and/or CO2 removal during medical procedures such as anaesthesia. It is difficult to provide the right level of oxygenation and/or CO2 removal. The time to pre-oxygenate and the highest achievable end-tidal oxygen level vary considerably between patients, application method and who is applying it.

**SUMMARY**

In accordance with a first aspect of the disclosure, there is provided a system for oxygenating a patient in relation to anaesthesia using high flow gas delivery comprising: a flow source, and a controller for determining oxygenation requirements of the patient before or during anaesthesia.

The controller may be adapted to control the flow and/or oxygen concentration of the high flow gas to assist oxygenation of the patient according to the oxygenation requirements.

The controller may be adapted to maintain the flow and/or oxygen concentration of the high flow gas.

The controller may be adapted to increase flow and/or oxygen concentration of the high flow gas to assist oxygenation of the patient if sufficient oxygenation has not occurred.

The controller may include a timer adapted to indicate the period of time over which gases have been delivered.

The controller may be adapted to determine if sufficient oxygenation has not occurred by monitoring one or more respiratory gases and/or monitoring the patient's blood oxygen saturation level.

The one or more respiratory gases may comprise oxygen and the system may comprise an oxygen gas analyser.

The controller may be adapted receive input indicating a first quantity of oxygen inhaled by the patient Oi and a second quantity of oxygen exhaled by the patient Oo, and the controller is configured to: control the flow source to deliver a first flow therapy, receive the input indicating Oi and Oo over at least one respiratory cycle, and control the flow source to continue delivering the first flow therapy or to deliver a second flow therapy on the basis of a function of the Oi and Oo.

The one or more respiratory gases may comprise nitrogen and the system may comprise a nitrogen gas analyser.

The controller may be adapted to receive input indicating a first quantity of nitrogen inhaled by the patient Ni and a second quantity of nitrogen exhaled by the patient No, and the controller is configured to: control the flow source to deliver a first flow therapy, receive input indicating Ni and No over at least one respiratory cycle, and control the flow source to continue delivering the first flow therapy or to deliver a second flow therapy on the basis of a function of the Ni and No.

**[0017]** In accordance with a second aspect of the disclosure, there is provided a method of oxygenating a patient in relation to anaesthesia using high flow gas delivery comprising determining oxygenation requirements of the patient before or during anaesthesia.

The method may further comprise controlling the flow and/or oxygen concentration of the high flow gas to assist oxygenation of the patient according to the oxygenation requirements.

The method may further comprise maintaining the flow and/or oxygen concentration of the high flow gas.

The method may further comprise increasing the flow and/or oxygen concentration of the high flow gas to assist oxygenation of the patient if sufficient oxygenation has not occurred.

Determining if sufficient oxygenation has not occurred may comprise monitoring one or more respiratory gases and/or monitoring the patient's blood oxygen saturation level.

The method may further comprise timing the period of time over which gases have been delivered.

The method may further comprise monitoring the one or more respiratory gases comprises monitoring oxygen.

The method may further comprise monitoring oxygen comprises receiving input indicating a first quantity of oxygen inhaled by the patient Oi and a second quantity of oxygen exhaled by the patient Oo, and: controlling the flow source to deliver a first flow therapy, receiving the input indicating Oi and Oo over at least one respiratory cycle, and controlling the flow source to continue delivering the first flow therapy or to deliver a second flow therapy on the basis of a function of the Oi and Oo.

The one or more respiratory gases may comprise nitrogen and the method may comprise monitoring nitrogen. Monitoring nitrogen may comprise receiving input indicating a first quantity of nitrogen inhaled by the patient Ni and a second quantity of nitrogen exhaled by the patient No, and: controlling the flow source to deliver a first flow therapy, receiving the input indicating Ni and No over at least one respiratory cycle, and controlling the flow source to continue delivering the first flow therapy or to deliver a second flow therapy on the basis of a function of the Ni and No.

**[0026]** In accordance with a third aspect of the disclosure, there is provided a system for oxygenating a patient in relation to anaesthesia using high flow gas delivery comprising: a flow source, and a controller for determining oxygenation requirements of the patient before anaesthesia when the patient is breathing.

The controller may be adapted to control the flow source to provide a high flow gas flow.

The controller may be adapted to control the flow source to provide an initial gas flow rate based on at least the BMI or any other patient parameters in combination with BMI.

The initial gas flow rate may be above 30 L/min.

Upon monitoring the transcutaneous O2 level, the controller may be adapted to increase oxygen concentration in the gas flow if the oxygen saturation level is lower than 99%, the transcutaneous O2 level is lower than 380mmHg, and the transcutaneous O2 level minus a predetermined value is lower than a previous value.

Upon monitoring the transcutaneous CO2 level, the controller may be adapted to increase oxygen concentration of the gas flow if the transcutaneous CO2 level is greater than 30mmHg and if the new transcutaneous CO2 level is greater than a previous saturation level plus a predetermined value.

Upon monitoring blood oxygen saturation level, the controller may be adapted to produce a warning if the blood oxygen saturation level has fallen.

Upon monitoring blood oxygen saturation and transcutaneous CO2, the controller may be adapted to indicate the end of the pre-oxygenation phase if the oxygen saturation is greater than 99% and the transcutaneous CO2 is equal to or less than 30mmHg.

In accordance with a fourth aspect of the disclosure, there is provided a system for oxygenating a patient in relation to anaesthesia using high flow gas delivery comprising: a flow source, and a controller for determining oxygenation requirements of the patient during anaesthesia when the patient is apnoeic.

The controller may be adapted to control the flow source to provide a high flow gas flow.

The controller may be adapted to control the flow source to provide an initial gas flow rate based on at least the BMI or any other patient parameters in combination with BMI such that flow rate is proportional to BMI.

The initial gas flow rate may be above 70 L/min.

Upon monitoring blood oxygen saturation level, the controller may be adapted to produce a warning if the blood oxygen saturation level is less than 92%.

Upon monitoring the blood oxygen saturation, the controller may be adapted to increase the flow of the gas flow if the average rate of change of blood oxygen saturation is negative, and if the rate of change of blood oxygen saturation is not increasing, and if the flow or 100L/minute or greater.

Upon monitoring the blood oxygen saturation, the controller may be adapted to increase the flow of the gas flow if the average rate of change of blood oxygen saturation is negative, if the flow is less than 100L/minute and if the rate of change of blood oxygen saturation is not increasing.

Upon monitoring the blood oxygen saturation, the controller may be adapted to warn the clinician if the average rate of change of blood oxygen saturation is negative, if the flow is 100L/minute or greater and if the oxygen concentration is 99% or greater.

Upon monitoring the blood oxygen saturation, the controller may be adapted to increase the flow and/or oxygen concentration of the gas flow if the average rate of change of blood oxygen saturation is negative and if the rate of change of blood oxygen saturation is increasing.

Upon monitoring the blood oxygen saturation, the controller may be adapted to decrease oxygen concentration of the gas flow if the average rate of change of blood oxygen saturation is zero or positive and if the average level of blood oxygen saturation is 99% or greater.

There is provided a method of oxygenating a patient in relation to anaesthesia using high flow gas delivery comprising determining oxygenation requirements of the patient before anaesthesia when the patient is breathing.

The method may further comprise controlling the flow source to provide a high flow gas flow.

The method may further comprise controlling the flow source to provide an initial gas flow rate based on at least the BMI or any other patient parameters in combination with BMI.

The initial gas flow rate may be above 30 L/min.

The method may further comprise controlling the oxygen concentration in the gas flow if the oxygen saturation level is lower than 99%, the transcutaneous O2 level is lower than 380mmHg, and the transcutaneous O2 level minus a predetermined value is lower than a previous value.

The method may further comprise increasing oxygen concentration of the gas flow if the transcutaneous CO2 level is greater than 30mmHg and if the new transcutaneous CO2 level is greater than a previous saturation level plus a predetermined value.

The method may further comprise, upon monitoring blood oxygen saturation level, producing a warning if the blood oxygen saturation level has fallen.

The method may further comprise, upon monitoring blood oxygen saturation and transcutaneous CO2, indicating the end of the pre-oxygenation phase if the oxygen saturation is greater than 99% and the transcutaneous CO2 is equal to or less than 30mmHg.

There is provided a method of oxygenating a patient in relation to anaesthesia using high flow gas delivery comprising determining oxygenation requirements of the patient during anaesthesia when the patient is apnoeic.

The method may further comprise controlling the flow source to provide a high flow gas flow.

The method may further comprise, controlling the flow source to provide an initial gas flow rate based on at least the BMI or any other patient parameters in combination with BMI such that flow rate is proportional to BMI.

The initial gas flow rate may be above 70 L/min.

The method may further comprise, upon monitoring blood oxygen saturation level, the controller may be adapted to produce a warning if the blood oxygen saturation level is less than 92%.

The method may further comprise, upon monitoring the blood oxygen saturation, increasing the flow of the gas flow if the average rate of change of blood oxygen saturation is negative, and if the rate of change of blood oxygen saturation is not increasing, and if the flow or 100L/minute or greater.

The method may further comprise, upon monitoring the blood oxygen saturation, increasing the flow of the gas flow if the average rate of change of blood oxygen saturation is negative, if the flow is less than 100L/minute and if the rate of change of blood oxygen saturation is not increasing.

The method may further comprise, upon monitoring the blood oxygen saturation, warning the clinician if the average rate of change of blood oxygen saturation is negative, if the flow is 100L/minute or greater and if the oxygen concentration is 99% or greater.

The method may further comprise, upon monitoring the blood oxygen saturation, increasing the flow and/or oxygen concentration of the gas flow if the average rate of change of blood oxygen saturation is negative and if the rate of change of blood oxygen saturation is increasing.

The method may further comprise, upon monitoring the blood oxygen saturation, decreasing oxygen concentration of the gas flow if the average rate of change of blood oxygen saturation is zero or positive and if the average level of blood oxygen saturation is 99% or greater.

Certain features, aspects and advantages of at least one of the configurations disclosed herein include the realization that a nasal cannula or other nasal interface can be used to deliver gases to a patient undergoing intubation or endoscopy. The delivery of gas therapy (e.g. high flow gas therapy) together with supplemental oxygen can substantially reduce or prevent the rise of blood carbon dioxide while maintaining blood oxygenation saturation levels within an acceptable range. The gas composition delivered using the gas therapy can be a function of, for example, the patient's blood oxygen saturation or nitrogen respiration ratio (e.g. ratio of expired nitrogen to inspiration nitrogen). If the flow is heated and humidified, the health of the patient's respiratory airway can preserved. Additionally, several systems, kits and/or arrangements are disclosed that may be useful for optimizing gas therapy for patients undergoing intubation or endoscopy.

Thus, in accordance with certain features, aspects and advantages of at least one of the embodiments disclosed herein, a respiratory therapy system is disclosed. The respiratory therapy system may comprise a flow source or generator adapted to provide a gas flow to a patient. The respiratory therapy system may comprise a sensor. In some configurations, the sensor may be adapted to measure a characteristic of the patient, such as but not limited to the patient's blood oxygen saturation. In some configurations, the sensor may be adapted to measure a characteristic of a respiratory gas. The sensor may be adapted to measure a first quantity of nitrogen inhaled by the patient Ni and a second quantity of nitrogen exhaled by the patient No. The respiratory therapy system may comprise a hardware controller. The hardware controller may be configured to control the flow generator to deliver a first flow therapy, receive the measured Ni and No and/or the measured blood oxygen saturation and/or other patient characteristics over at least one respiratory cycle, and control the flow generator to continue delivering the first flow therapy or to deliver a second flow therapy on the basis of a function of the measured Ni and No and/or measured blood oxygen saturation.

In some configurations, if the No is less than or equal to the Ni¬ plus a threshold nitrogen variance Np, the flow generator may be controlled to deliver the second flow therapy.

In some configurations, if the ratio of No:Ni is less than or equal to a predetermined nitrogen ratio Np_r, the flow generator may be controlled to deliver the second flow therapy.

In some configurations, different concentrations of gases may be delivered in the second flow therapy than in the first flow therapy.

In some configurations, during preoxygenation the first flow therapy may comprise delivery of a first gas composition comprising a first concentration of ambient gas Ga1 and a first concentration of oxygen Go1, and the second flow therapy may comprise delivery of a second concentration of ambient gas G¬a2 and a second concentration of oxygen Go2. In some such configurations, Go1 ¬may be greater than Go2. In some such configurations, the ratio of Go1 to (Ga1+Go1) may be greater than the ratio of Go2 to (Ga2+Go2).

Additionally, in accordance with certain features, aspects and advantages of at least one of the embodiments disclosed herein, a respiratory therapy kit is disclosed. The respiratory therapy kit may comprise a patient interface, a humidifier comprising an inlet adapted to be connected to a flow generator, and a conduit adapted to fluidly communicate or allow for fluid communication between the humidifier and the patient interface. The patient interface, the conduit and/or at least a part of the humidifier may be integrally moulded or in the form of a single part.

In some configurations, the humidifier may further comprise a fluid reservoir. In some such configurations, the respiratory therapy kit may further comprise a heating device. The heating device may comprise a chemical heater adapted to heat fluid in the fluid reservoir. The heating device may comprise a manually actuatable switch configured to activate the chemical heater.

In some configurations, the patient interface may comprise a port configured to accept a drug delivery device. In some such configurations, the respiratory therapy kit may comprise a drug delivery device adapted to be located in the port. The drug delivery device may comprise a manually actuatable metering mechanism configured to allow for the release of medication.

Additionally, in accordance with certain features, aspects and advantages of at least one of the embodiments disclosed herein, a respiratory therapy system is disclosed. The respiratory therapy system may comprise a respiratory therapy apparatus. The respiratory therapy apparatus may comprise a flow generator, a humidifier, and/or an integrated flow generator/humidifier apparatus. The respiratory therapy system may also comprise a patient interface configured to be removably attachable to a conduit, and a conduit adapted to extend between the respiratory therapy apparatus and the patient interface. The respiratory therapy apparatus and the conduit may be integrally moulded or be in the form of a single part.

In some configurations, the respiratory therapy system may further comprise a disinfection arrangement adapted to disinfect at least a section of the conduit. In some such configurations, the respiratory therapy system may further comprise a hardware controller configured to control the disinfection arrangement, wherein the hardware controller controls the disinfection arrangement dependent on whether or not the patient interface is attached to the conduit. In some such configurations, the respiratory therapy system may further comprise a hardware controller configured to control the disinfection arrangement, wherein the hardware controller is configured to activate the disinfection arrangement after removal of the patient interface from the conduit. In the above configurations or as demonstrated or implied elsewhere in this disclosure, the respiratory therapy system may further comprise a flow or pressure sensor. The hardware controller may be configured to, in use, utilize signals from the flow or pressure sensor or values derived therefrom (e.g. from the signals of the flow sensor or of the pressure sensor) to determine if the patient interface is or is not attached to the conduit. The signals may be used to, for example, help control the on/off state and/or the intensity of disinfection of the disinfection arrangement.

In some configurations, the respiratory therapy system may further comprise a one-way valve adapted to prevent the flow of exhaled gases into the conduit. The one-way valve may be positioned within the conduit.

The present disclosure relates to methods, apparatus and systems for estimating pre-oxygenation parameters for these patients. In particular, the present methods, apparatus and systems may be used to determine a sufficient level of pre-oxygenation, and/or predict the time required for the patient to be sufficiently pre-oxygenated and/or monitor the level of pre-oxygenation of the patient.

In accordance with certain features, aspects and advantages of at least one of the embodiments disclosed herein, a method is disclosed for estimating and/or determining one or more pre-oxygenation parameters for a patient undergoing pre-oxygenation therapy. The method disclosed comprises one or more of the following steps:

Measuring and/or tracking the concentration of one or more respiratory gases in the patient's expired gas,

Measuring and/or tracking the concentration of one or more respiratory gases in the patient's blood (blood saturation),

measuring and/or tracking the concentration of a tracer introduced to the patient's respiratory tract

Measuring and/or tracking one or more physical properties of the patient's expired gas.

In some configurations, the pre-oxygenation parameters comprise:

a) the time until a patient undergoing a pre-oxygenation procedure has been sufficiently pre-oxygenated,

b) the likelihood of a pre-oxygenation procedure achieving a target end-tidal oxygen level for a patient,

c) the end-tidal oxygen level for a patient undergoing a pre-oxygenation procedure,

d) if a patient has been sufficiently pre-oxygenated during a pre-oxygenation procedure,

e) changes in pre-oxygenation level of a patient undergoing a pre-oxygenation procedure.

In some configurations, the respiratory gas is one or more of oxygen, nitrogen, and carbon dioxide.

In some configurations, the step of measuring and/or tracking the concentration of one or more respiratory gases in the patient's expired gas is performed using a mass spectrometer.

In some configurations, the step of measuring and/or tracking the concentration of one or more respiratory gases in the patient's expired gas is performed using a gas analyser.

In some configurations, the gas analyser is one or more of an oxygen gas analyser, a nitrogen gas analyser, and a carbon dioxide analyser.

In some configurations, the step of measuring and/or tracking the concentration of one or more respiratory gases in the patient's blood is performed via pulse oximetry.

In some configurations, the tracer is introduced to the patient's respiratory tract via inhalation prior to delivery of the pre-oxygenation therapy.

In some configurations, the tracer is introduced to the patient's respiratory tract via inhalation during an initial warm-up phase of the pre-oxygenation therapy.

In some configurations, the tracer is one or more of nitrogen, helium, argon and sulfur-hexafluoride.

In some configurations, the concentration of the tracer is measured and/or tracked using one or more of:

a) an absorption spectrometer

b) a non-dispersive optical gas analyser

c) infrared gas analyser

d) an ultrasonic gas analyser

e) a mass spectrometer

In some configurations, the step of measuring and/or tracking the one or more physical properties of the patient's expired gas is used to estimate the fractional concentrations of one or more respiratory gases in the expired gas.
In some configurations, said one or more physical properties comprises:

a) density,

b) viscosity,

c) acoustic response.

In some configurations, said one or more measuring and/or tracking steps is performed continuously.
In some configurations, said continuous measurement and/or tracking is performed until the patient is sufficiently pre-oxygenated.
In some configurations, sufficient pre-oxygenation is indicated by a sustained end-tidal oxygen concentration and/or blood oxygen saturation level greater than 70%, preferably 87%.
In some configurations, sufficient pre-oxygenation is indicated by a sustained end-tidal oxygen concentration and/or blood oxygen saturation level greater than 95%.
In some configurations, sufficient pre-oxygenation is indicated by substantially steady-state concentration(s) and/or blood saturation level(s) of one or more respiratory gases and/or tracer.
In some configurations, the method further comprises the steps of:

a) measuring and/or estimating the rate of change of the patient's expired concentration and/or blood saturation of said one or more respiratory gases and/or tracer
b) fitting the rate of change to a non-linear model
c) determining a time constant for said model
d) multiplying the time constant with a treatment factor to estimate the time until sufficient pre-oxygenation may be achieved.

In some configurations, the non-linear model is an exponential curve.
In some configurations, the treatment factor is four.
In some configurations, said time until sufficient pre-oxygenation is further multiplied by a safety factor.
In some configurations, the time constant is the time taken for the concentration of nitrogen in the expired gas to drop to substantially 37% of the concentration of nitrogen in the first measurement of expired gas.
In some configurations, the time constant is the time taken for the concentration of tracer in the expired gas to drop to substantially 37% of the concentration of tracer in the first measurement of expired gas.
In some configurations, the time constant is the time taken for the concentration of oxygen in the expired gas to increase by substantially 63% of the difference between the desired level (EtO2 = 87%) and the concentration of oxygen in the first measurement of expired gas. If the first EtO2 measurement was 50%, for example, then the time constant would be the time taken for the oxygen concentration to increase by (87 - 50)*0.63 percent.
In some configurations, the time constant is the time taken for the blood saturation of oxygen to increase by substantially 63% of the difference between the desired level (95% or greater, preferably 100%) and the patient's blood saturation of oxygen prior to or at the start of the pre-oxygenation procedure. For example, if there is a target final saturation level of 100% and the initial saturation level was 90%, then the time constant would be the time taken for the percentage saturation to increase from 90% to (90 + (100 - 90)*0.63) = 96.3%
In some configurations, the time constant is the quotient of dividing the patient's functional residual capacity by the patient's alveolar ventilation.
In some configurations, pre-oxygenation is delivered via high flow therapy.
In some configurations, pre-oxygenation is delivered via a sealed or unsealed nasal interface, preferably a nasal cannula.
In some configurations, apparatus is disclosed configured for estimating and/or determining one or more pre-oxygenation parameters, comprising one or more sensors configured to:

a) measure and/or track the concentration of one or more respiratory gases in the patient's expired gas and/or

b) measure and/or track the concentration of one or more respiratory gases in the patient's blood (blood saturation) and/or

c) measure and/or track the concentration of a tracer introduced to the patient's respiratory tract and/or

d) measure and/or track one or more physical properties of the patient's expired gas.

In some configurations, the pre-oxygenation parameters comprise:

a) the time until a patient undergoing a pre-oxygenation procedure has been sufficiently pre-oxygenated,

b) the likelihood of a pre-oxygenation procedure achieving a target end-tidal oxygen level for a patient,

c) the end-tidal oxygen level for a patient undergoing a pre-oxygenation procedure,

d) if a patient has been sufficiently pre-oxygenated during a pre-oxygenation procedure,

e) changes in pre-oxygenation level of a patient undergoing a pre-oxygenation procedure.

In some configurations, the apparatus further comprises a processor configured to:

a) fit the rate of change of the patient's expired concentration and/or blood saturation of said one or more respiratory gases and/or tracer to a non-linear model,

b) determine a time constant for said model,

c) multiply the time constant with a treatment factor to estimate the time until sufficient pre-oxygenation may be achieved,

d) indicate when the patient has been sufficiently pre-oxygenated.

In some configurations, the apparatus further comprises a timer configured to indicate the estimated time remaining until the patient is sufficiently pre-oxygenated.

In some configurations, the apparatus further comprises a control interface or a connection to a control interface of a pre-oxygenation therapy apparatus or system providing pre-oxygenation therapy to said patient.

As relatively high gas delivery flow rates may be used with the embodiments or configurations described herein, the gases being supplied or delivered to the user or patient may be delivered to different parts of the user's or a patient's airway.

Delivering oxygen through high flow therapy e.g. flow rates above 10L/min achieves pre-oxygenation to higher levels faster than traditional methods using a facemask with an oxygen reservoir, non-rebreather masks, self-inflating bag-valve-masks with or without 1-way valves. Other high flow rates are contemplated.

For example, according to those various embodiments and configurations described herein, a flowrate of gases supplied, such as a high flow rate, provided to an interface or via a system, such as through a flowpath, may comprise a gas flow rate of greater than 15 L/min (Liters per minute), greater than or equal to about 20 L/min, greater than or equal to about 30 L/min, greater than or equal to about 40 L/min, greater than or equal to about 50 L/min, greater than or equal to about 60 L/min, greater than or equal to about 70 L/min, greater than or equal to about 80 L/min, greater than or equal to about 90 L/min, greater than greater than or equal to about 100 L/min, greater than about or equal to 110 L/min, greater than about or equal to 120 L/min, greater than about or equal to 130 L/min, greater than about or equal to 140 L/min or up to about 150 L/min. In certain embodiments, useful ranges of a high gas flow can be selected between any of the aforementioned flow rates including but not limited to from about 40 L/min to about 80 L/min, from about 50 L/min to about 80 L/min, from about 70 L/min to about 100 L/min, about 70 L/min to about 80 L/min, about 100 L/min to about 150 L/min and about greater than 15 L/min to about 150 L/min and about 30 L/min to about 150 L/min. These flow rates can be provided using a patient interface and in certain embodiments through a nasal interface.

Such relatively high flowrates of gases may assist in providing the supplied gases into a user's airway, or to different parts of a user's airway, for example such flowrates may allow for a delivery of such gases to the upper or lower airway regions. Upper airway region typically includes the nasal cavity, pharynx and larynx, while the lower airway region typically includes the trachea, primary bronchi and lungs.

It should be understood that alternative embodiments may comprise any or all combinations of two or more of the parts, elements or features illustrated, described or referred to in this specification.

In accordance with at least one embodiment disclosed herein is a method of oxygenating a patient in relation to anaesthesia using high flow gas delivery comprising determining oxygenation requirements of the patient before or during anaesthesia.

In some configurations determining oxygenation requirements comprises one or more of:

- receiving input indicative of risk assessment

- receiving input indicative of oxygenation requirements of the patient;

- receiving input relating to patient physiology and using the input to determine oxygenation requirements of the patient, optionally wherein the input could be one or more of patient:

  - age,

  - weight,

  - height,

  - body fat measure (e.g. percentage) or body fat distribution)

  - BMI,

  - lung volume,

  - metabolic rate;

- receiving input relating to pre-existing patient conditions and using the input to determine oxygenation requirements of the patient (e.g. susceptibility to airway obstruction such as OSA risk/OSA index;

- sensing physiological parameters of the patient and using that to determine oxygenation requirements of the patient;

- monitoring $O_2$ supply to and/or $CO_2$ removal from the patient and from that determining oxygenation requirements;

- ascertaining limits on apparatus delivering the high flow gas and using that to determine oxygenation requirements;

- determining the stage of anaesthesia and using that to determine oxygenation requirements;

- determining the expected or monitoring the actual duration of one or more stages of anaesthesia and using that to determine oxygenation requirements;

- receiving input of actual high flow gas parameter settings required for the oxygenation requirement.

Determining oxygenation requirements comprises determining oxygen concentration and/or oxygen flow rate.

In some configurations the stages of anaesthesia comprise one or more of:

- pre-anaesthesia where the patient is breathing (pre-oxygenation),

- during anaesthesia where the patient is apnoeic.

In some configurations detecting the stage of anaesthesia by detecting breathing pressure to determine if the patient is: a) breathing and in the pre-oxygenation state, or b) not breathing and in the apnoeic stage.

In some configurations the method further comprises detecting the stage of anaesthesia by detecting the expired $CO_2$ to determine if the patient is: a) breathing and in the pre-oxygenation state, or b) not breathing and in the apnoeic stage.

In some configurations monitoring O2 supply and/or CO2 removal comprises monitoring one or more of:

- expired O2, CO2,

- transcutaneous O2, CO2,

- blood gases,

- SpO2.

In some configurations the method further comprises controlling one or parameters of the high flow gas to assist oxygenation of the patient according to the oxygenation requirements.

In some configuration controlling one or more parameters of the high flow gas comprises controlling one or more of:

- flow rate of gas (such as flow rate of oxygen)

- volume of gas delivered

- pressure of gas

- composition and/or concentration of gas

In some configurations the method further comprises, upon monitoring O2 supply and/or CO2 removal, increasing flow and/or oxygen concentration if:

- SpO2 decreases past 92%,

- End tidal CO2 increases

- The SpO2 decreases at a rate greater than a threshold rate, for example 1% per minute.

In some configurations upon determining a short duration of one or more stages of anaesthesia, increasing the flow and/or oxygen concentration.

In some configurations the high flow gas is delivered and the oxygenation requirements are determined using a high flow therapy apparatus.

In accordance with at least one embodiment disclosed herein is a system for oxygenating a patient in relation to anaesthesia using high flow gas delivery comprising: a flow source, and a controller for determining oxygenation requirements of the patient before or during anaesthesia.

In some configurations determining oxygenation requirements comprises one or more of:

- receiving input indicative of risk assessment

- receiving input indicative of oxygenation requirements of the patient;

- receiving input relating to patient physiology and using the input to determine oxygenation requirements of the patient, optionally wherein the input could be one or more of patient:

  - age,

  - weight,

  - height,

  - body fat measure (e.g. percentage)

  - BMI,

- lung volume,

- metabolic rate;

- receiving input relating to pre-existing patient conditions and using the input to determine oxygenation requirements of the patient;

- sensing physiological parameters of the patient and using that to determine oxygenation requirements of the patient;

- monitoring O2 supply to and/or CO2 removal from the patient and from that determining oxygenation requirements;

- ascertaining limits on apparatus delivering the high flow gas and using that to determine oxygenation requirements;

- determining the stage of anaesthesia and using that to determine oxygenation requirements;

- determining the expected or monitoring the actual duration of one or more stages of anaesthesia and using that to determine oxygenation requirements;

- receiving input of actual high flow gas parameter settings required for the oxygenation requirement.

In some configurations the stages of anaesthesia comprise one or more of:

- pre-anaesthesia where the patient is breathing (pre-oxygenation),

- during anaesthesia where the patient is apnoeic.

In some configurations the controller further detects the stage of anaesthesia by detecting breathing pressure to determine if the patient is: a) breathing and in the pre-oxygenation state, or b) not breathing and in the apnoeic stage.
In some configurations the controller further detects the stage of anaesthesia by detecting the expired CO2 to determine if the patient is: a) breathing and in the pre-oxygenation state, or b) not breathing and in the apnoeic stage.
In some configurations monitoring O2 supply and/or CO2 removal comprises monitoring one or more of:

- expired O2, CO2,

- transcutaneous O2, CO2,

- blood gases,

- SpO2.

In some configurations the controller further controls one or parameters of the high flow gas to assist oxygenation of the patient according to the oxygenation requirements.
In some configurations controlling one or more parameters of the high flow gas comprises controlling one or more of:

- flow rate of gas (such as flow rate of oxygen)

- volume of gas delivered

- pressure of gas

- composition and/or concentration of gas

In some configurations the method further comprises the controller, upon monitoring O2 supply and/or CO2 removal, increasing flow and/or oxygen concentration if:

- SpO2 decreases past 92%

- End tidal CO2 increases

- The SpO2 decreases at a rate greater than a threshold rate, for example 1% per minute.

In some configurations upon determining a short duration of one or more stages of anaesthesia, the controller increases the flow and/or oxygen concentration.

In accordance with at least one embodiment disclosed herein is a system for oxygenating a patient in relation to anaesthesia using high flow gas delivery comprising: a flow source, one or more sensors to monitor parameters in order to establish: 1) oxygenation requirements, 2) stage of anaesthesia treatment 3), and/or O2 and CO2 changes, and a controller connected to the sensors for determining oxygenation requirements , stage of anaesthesia and/or O2, CO2 changes of the patient before or during anaesthesia.

In accordance with at least one embodiment disclosed herein is a system for oxygenating a patient in relation to anaesthesia using high flow gas delivery comprising: a flow source, one or more sensors to monitor parameters in order to establish: 1) oxygenation requirements, 2) stage of anaesthesia treatment 3), and/or O2 and CO2 changes, and a controller connected to the sensors and configured to determine changes in the parameters and modify one or more of the settings of the flow source and/or or control other parts of the system in order to compensate for the changes and allow for change in therapy dose.

In some configurations the information related to the patient can be accessed remotely from a database. Further the system can automatically provide therapy settings based on stored patient information. Also a clinician can remotely operate the system.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

The invention consists in the foregoing and also envisages constructions of which the following gives examples only.

## BRIEF DESCRIPTION OF THE DRAWINGS

Specific embodiments and modifications thereof will become apparent to those skilled in the art from the detailed description herein having reference to the figures that follow, of which:

Figure 1 shows a schematic diagram of a respiratory therapy system.

Figure 2 shows a schematic diagram of a respiratory therapy system in use.

Figures 3A-3B show flow charts illustrating a respiratory therapy method.

Figure 4 shows a schematic diagram of a respiratory therapy system.

Figure 5 shows a flow chart illustrating a respiratory therapy method

6 shows a flow chart illustrating a respiratory therapy method.

Figure 7 shows a respiratory therapy kit.

Figure 8 illustrates an apparatus/system for oxygenating a patient with high flow gas in relation to anaesthesia.

Figure 9 illustrates a method for oxygenating a patient with high flow gas in relation to anaesthesia.

Figure 10 illustrates a method of determining a stage of anaesthesia.

Figure 11 illustrates airways of a patient.

Figure 12a shows an exemplary exponential wash-out curve,

Figure 12b shows an exemplary exponential wash-in curve,

Figure 12c shows an exemplary trace obtained from continuous nitrogen measurement.

## DETAILED DESCRIPTION

The present disclosure relates to respiratory gas therapy systems, apparatus, kits and methods for treating patients undergoing intubation or endoscopy. The present disclosure also relates to apparatus, systems, and methods for estimating pre-oxygenation parameters for a patient undergoing pre-oxygenation respiratory gas therapy. The present disclosure also particularly relates to determining oxygenation requirements for a patient in relation to anaesthesia, and providing gas flow with parameters meeting the oxygenation requirements.

It is an object of certain embodiments disclosed herein to provide a method or apparatus which will go at least some way towards addressing the foregoing problems or which will at least provide the industry with a useful choice. It is also an object of one or more of the disclosed embodiments to determine and/or provide oxygenation requirements of a patient in relation to anaesthesia and/or to at least provide the public with a useful choice.

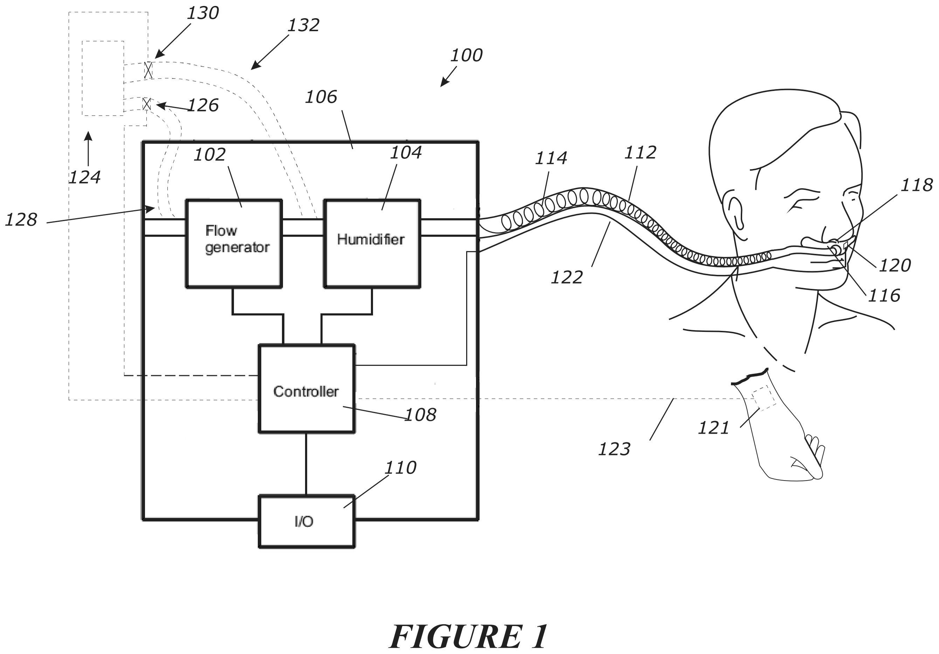

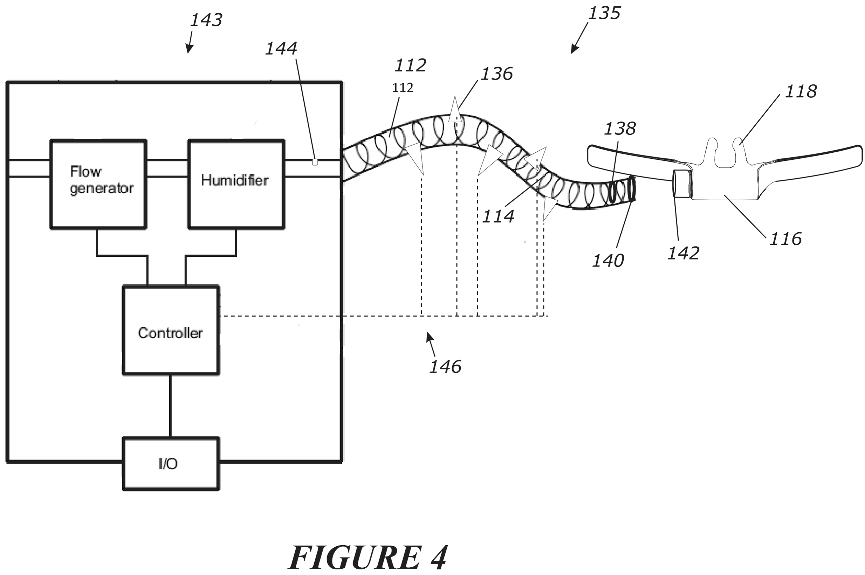

For the sake of convenience, certain features present or annotated with reference numerals in some figures of the present disclosure are not shown or annotated with reference numerals in other figures of the present disclosure. For example, Figure 1 illustrates a supplementary gas source 124, whereas Figure 4 does not, and likewise Figure 4 shows a one-way valve 138, whereas Figure 1 does not. However, unless the context clearly requires otherwise, these omissions should not be interpreted to mean that features omitted from the drawings of one figure could not be equally incorporated or implemented in the configurations of the disclosed methods, apparatus and systems related to or embodied in other figures. For example, in some configurations, Figure 1 may be interpreted as incorporating the one-way valve 138 disclosed in Figure 4 and in the associated passages of the specification, and similarly, in some configurations, Figure 4 may be interpreted as incorporating the supplementary gas source 124 disclosed in Figure 1 and in the associated passages of the specification. Conversely, unless the context clearly requires otherwise, it should not be assumed that the presence of certain features in some figures of the present disclosure mean that the disclosed methods, apparatus, kits and systems related to or embodied in these figures must necessarily include these features and/or the use of such features.

The present disclosure relates to methods, systems and/or apparatus to assist medical professionals with pre-oxygenation procedures, to reduce the risk of the patient becoming hypoxic or hypoxemic in the subsequent intubation procedure. The disclosure herein may provide for particular usefulness in medical procedures where a patient is sedated or for patients where respiratory drive is compromised or where a patient's respiratory drive has been purposely reduced.

The present methods, systems and/or apparatus are particularly useful for medical procedures where a patient is sedated, as they often stop spontaneous breathing. Additionally, the present methods, systems and/or apparatus may be useful for patients whose respiratory drive has been compromised or purposely reduced.

A continuous supply of oxygen is essential to sustain healthy respiratory function during anaesthesia. When this supply is compromised, hypoxia can occur. During anaesthesia, the patient is monitored to ensure this does not happen. If oxygen supply is compromised the clinician stops the medical procedure and facilitates oxygen supply. This can be achieved for example by manually ventilating the patient through bag mask systems.

In an improvement, (high) flow gas (e.g. oxygen or a mix of oxygen and one or more other gases) can be delivered to a patient to reduce the risk of hypoxia. This high flow gas can be provided prior to anaesthesia (pre-oxygenation) while the patient is still breathing, or during anaesthesia or anaesthesia procedures, including when the patient might be apnoeic. This gas flow might be provided at a constant flow rate to deliver the "dose" oxygen required (patient oxygen requirement) to avoid hypoxia. This dose can also be referred to as the required "therapy" or "support". The dose relates to the one or more parameters of the high flow gas being delivered, and an optimal or required dose relates to the high flow gas parameters that provide a patient with their oxygen requirements. For example, the parameters might be (although are not limited to) one or more of:

- flow rate of gas (such as flow rate of oxygen and including variable flow or a repeating flow waveform)

- volume of gas delivered

- pressure of gas

- composition and/or concentration of gas (the composition may be air, a mixture of nitrogen and oxygen, oxygen only, or a variable concentration)

In general terms, the dose/oxygen requirements are determined before anaesthesia and/or during (e.g. thorough continuous or periodic monitoring) anaesthesia; and then the parameters of the high gas flow are altered accordingly (manually or automatically) to provide the required oxygenation to the patient. It should be noted that reference to "anaesthesia" and its stages throughout this specification can refer to actual anaesthesia, and the period prior to anaesthesia (such as the pre-oxygenation stage).

With reference to the non-limiting exemplary embodiment shown in Figure 1, a respiratory therapy system/-apparatus 100 is shown. The system/apparatus 100 may be an integrated or separate component based arrangement, generally shown by the solid box of figure 1. Hereinafter the system/apparatus will be referred to as system, but this should not be considered limiting. The system 100 comprises a flow source or flow generator 102. The flow source 102 provides a high flow gas such as oxygen, or a mix of oxygen and one or more other gases. The flow source could be an in-wall supply of oxygen, a tank of oxygen, a tank of other gas and/or a high flow therapy apparatus. The flow source is shown as part of the system 100, although in the case of an external oxygen tank or in-wall source, it may be considered a separate component. The flow source 102 provides a (preferably high) flow of gas 153 that can be delivered to a patient 156 via a delivery conduit 154, and patient interface 116(such as a nasal cannula). A humidifier 104 can optionally be provided between the flow source and the patient to provide humidification of the delivered gas.

The flow source 102 is configured to draw in ambient air or air outside of the system 100 and pass the air to the humidifier 104. In some configurations, the flow source 102 may comprise some other gas generation means. For example, in some configurations, the flow source 102 may comprise a container of compressed air or another gas and a valve arrangement adapted to control the rate at which gases leave the container. As another example, in some configurations, the flow source 102 may comprise an oxygen concentrator. In some configurations, the flow source 102 may be adapted to deliver a high flow therapy.

"High flow therapy", "High gas flow" or "high gas flows" as used herein is defined as the volumetric movement of a portion/parcel of gas or mixtures of gases into the patient's airways at rates exceeding the fraction of inspired oxygen requirements at peak inspiratory flow demand. In particular, in one embodiment, high gas flow (or high gas flows) refers to gas flow rate of greater than 15 L/min (Liters per minute), greater than or equal to about 20 L/min, greater than or equal to about 30 L/min, greater than or equal to about 40 L/min, greater than or equal to about 50 L/min, greater than or equal to about 60 L/min, greater than or equal to about 70 L/min, greater than or equal to about 80 L/min, greater than or equal to about 90 L/min, greater than greater than or equal to about 100 L/min, greater than about or equal to 110 L/min, greater than about or equal to 120 L/min, greater than about or equal to 130 L/min, greater than about or equal to 140 L/min or up to about 150 L/min. In certain embodiments, useful ranges of a high gas flow can be selected between any of the aforementioned flow rates including but not limited to from about 40 L/min to about 80 L/min, from about 50 L/min to about 80 L/min, from about 70 L/min to about 100 L/min, about 70 L/min to about 80 L/min, about 100 L/min to about 150 L/min and about greater than 15 L/min to about 150 L/min and about 30 L/min to about 150 L/min. These flow rates can be provided using a patient interface and in certain embodiments through a nasal interface.

In any of the embodiments described herein, the high gas flow can be humidified. In some configurations the gas flow may be humidified to contain greater than 10 mg/L of water, or greater than 20 mg/L, or greater than 30 mg/L, or up to 44 mg/L. In some configurations the gas flow may be heated to 21 °C to 42 °C, or 25 °C to 40 °C, or 31 °C to 37 °C, or about 31 °C, or about 37 °C.

The system 100 also comprises a housing 106 that at least partially houses both the flow source 102 and the humidifier 104 (e.g. the system 100 may comprise an integrated flow source/humidifier apparatus), although in some configurations the flow source 102 and humidifier 104 may have separate housings. A controller 108 is shown to be in electronic communication with the flow source 102 and the humidifier 104, although in some configurations the controller 108 might only communicate with the flow source 102 or the humidifier 104. The controller 108 may comprise a microcontroller or some other architecture configured to direct the operation of controllable components of the system 100, including but not limited to the flow source 102 and/or the humidifier 104. An input/output module 110 (such as a display and/or input device) is shown to be in electronic communication with the controller 108. The input device is for receiving information from a user (e.g. clinician or patient) that can be used for determining oxygenation requirements. The input/output module 110 may be configured to allow a user to interface with the controller 108 to facilitate the control of controllable components of the system 100, including but not limited to the flow source 102 and/or the humidifier 104. In some configurations the gas flow may be humidified to contain greater than 10 mg/L of water, or greater than 20 mg/L, or greater than 30 mg/L, or up to 44 mg/L. In some configurations the gas flow may be heated to 21 °C to 42 °C, or 25 °C to 40 °C, or 31 °C to 37 °C, or about 31 °C, or about 37 °C. The input/output module 110 might comprise, for example, one or more buttons, knobs, dials, switches, levers, touch screens, speakers, displays and/or other input or output peripherals that a user might use to view data and/or input commands to control components of the system 100

As further shown in Figure 1, a supplementary gas source 124 may be used to deliver gas to a patient. The supplementary gas source 124 may be configured to deliver one or more supplementary gases including but not limited to oxygen (O2), carbon dioxide (CO2), nitrogen (N2), nitrous oxide (NO), and/or heliox. The supplementary gas source 124 may deliver the one or more supplementary gases via a first supplementary gas conduit 128 to a location upstream of the flow source 102, and/or may deliver the one or more supplementary gases via a second supplementary gas conduit 132 to a location downstream of the flow source 102 and/or upstream of the humidifier 104. One or more supplementary flow valves 126, 130 may be used to control the rates at which the one or more supplementary gases can flow from the supplementary gas source 124 and through the first and/or second supplementary gas conduits 128, 132. One or more of the supplementary flow valves 126, 130 may be in electronic communication with the controller 108, which may in turn control the operation and/or state of the one or more of the supplementary flow valves 126, 130.

As shown in Figure 1, a conduit 112 extending from the humidifier 104 links the humidifier 104 to a patient interface. The conduit 112 comprises a conduit heater 114 adapted to heat gases passing through the conduit 112, although in some configurations the conduit heater 114 may not be present. The patient interface may be a sealed or unsealed patient interface. Preferably, a nasal interface, and more preferably unsealed nasal cannula may be used. The patient interface is shown to be a nasal cannula 116, although it should be understood that in some configurations, other patient interfaces may be suitable. For example, in some configurations, the patient interface may comprise a sealing or non-sealing interface, and may comprise a nasal mask, an oral mask, an oro-nasal mask, a full face mask, a nasal pillows mask, a nasal cannula, an endotracheal tube, a combination of the above or some other gas conveying system. The nasal cannula 116 comprises a pair of nasal delivery elements 118 adapted to be fitted into the nares of a patient in a non-sealing manner, although other configurations, such as but not limited to having only a single non-sealing nasal delivery element 118 or having a combination of a sealing nasal delivery element 118 and a non-sealing nasal delivery element 118, may be used.

As shown the nasal cannula 116 also comprises a sensing module 120 adapted to measure a characteristic of gases passing through the nasal cannula 116, although the sensing module 120 could be positioned and adapted to measure the characteristics of gases at or near other parts of the system 100. For example, in some configurations, the sensing module 120 could be positioned in the nasal cannula 116 or upstream of the nasal cannula 116 (in some configurations proximal to the flow source 102). The sensing module 120 may comprise one or more sensors adapted to measure various characteristics of gases, including but not limited to pressure, flow rate, temperature, absolute humidity, relative humidity, enthalpy, gas composition, oxygen concentration, carbon dioxide concentration, and/or nitrogen concentration. For example, the sensing module may comprises one or more gas analyzers. Gas properties determined by the sensing module 120 may be utilized in a number of ways, including but not limited to closed loop control of parameters of the gases. For example, in some configurations flow rate data taken by a sensing module 120 may be used to determine the respiratory cycle of the patient to facilitate the delivery of flow in synchronicity with portions of the respiratory cycle. The sensing module 120 communicates with the controller 108 over a first data line 122. The first data line 122 may comprise a wired data communication connection such as but not limited to a data cable, or a wireless data communication connection such as but not limited to WiFi, Bluetooth, or NFC. Optionally, in some configurations, both power and data may be communicated over the same first data line 122. For example, the sensing module 120 may comprise a modulator that may allow a data signal to be 'overlaid' on top of a power signal. Optionally, the data signal may be superimposed over the power signal and the combined signal may be demodulated before use by the controller 108.

Additionally as shown, a physiological sensor module 121 may be present. The physiological sensor module 121 may be configured to detect various characteristics of the patient or of the health of the patient, including but not limited to heart rate, EEG signal, EKG/ECG signal, blood oxygen concentration, blood oxygen saturation (via, for example, a pulse oximeter), blood $CO_2$ concentration, transcutaneous $CO_2$ ($TcCO_2$) and/or blood glucose. The physiological sensor module 121 may comprises a gas analyser. Similarly, the physiological sensor module 121 may communicate with the controller 108 over a second data line 123. The second data line 123 may comprise wired or wireless data communication connections similarly to the first data line 122, and both power and data may be communicated similarly. The physiological sensor module 121 may be used, for example, to determine the oxygenation of the patient such that a physician may know when to begin the process of intubating the patient or performing an endoscopic procedure on the patient. In some such configurations, a pulse oximeter may be used to determine the blood oxygen saturation of the patient. If the blood oxygen saturation increased to meet or exceed a threshold blood oxygen saturation level $T_b$, the system 100 may output an indication or alarm to a physician (via, for example, an output module of a user interface) indicating that the physician should begin the process of intubation or endoscopy.

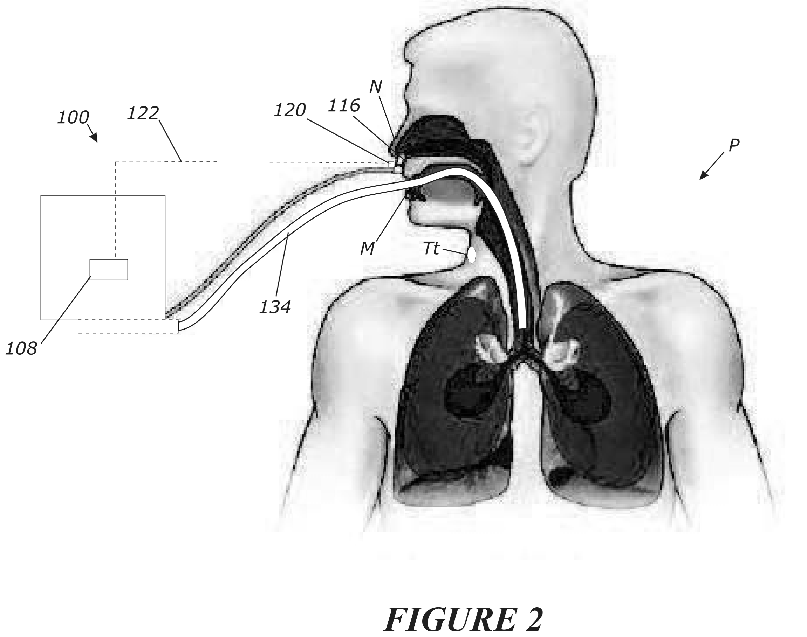

With reference to the non-limiting exemplary embodiment shown in Figure 2, use of a respiratory therapy system 100 is shown. As shown the system 100 can be used to deliver a gas therapy to a patient intubated with a tube 134 or being examined with an endoscope 134. In some cases, it can be helpful to ventilate a patient by using a nasal cannula 116 to propel gases through the nares N, allowing a physician to focus on completing an intubation procedure via the mouth M (or in some configurations, via the trachea Tt) without the inconvenience of having to interrupt the intubation to deliver additional concentrated oxygen gas via reapplication of a face mask (or in some configurations, of a tracheal mask). Additionally, the build-up of carbon dioxide that can occur when attempting to intubate a patient can be minimized if a high flow gas therapy is delivered to minimize anatomical dead space.

In some cases, delivering a high flow gas therapy at a relatively high temperature, for example between about 21°C and about 43°C, or between about 24°C and about 43°C, or between about 27°C and about 43°C, or between about 30°C and about 43°C, or between about 33°C and about 43°C, or between about 37°C and about 43°C, and/or delivering a high flow gas therapy at a relatively high humidity, for example between about 24mg/L and about 44mg/L, or between about 29mg/L and about 44mg/L, or between about 34mg/L and about 44mg/L, or between about 39mg/L and about 44mg/L, or about 44mg/L, can be beneficial for keeping a patient's airways relatively moist and healthy during an intubation process or an upper endoscopy, particularly if the process or endoscopy is prolonged due to, for example, a difficult airway. The high flow therapy may be continuously or discontinuously be delivered (at, for example, the flow rates and oxygen concentrations described above or elsewhere in this disclosure) during processes of intubation or endoscopy to prolong the apneic window during such processes.

When handling a patient that is to undergo intubation, particularly if the patient is or will be incapable of spontaneous respiration (for example, when sedated with a general anesthetic agent) and will be ventilated using gas therapy (e.g. high flow therapy through, for example, a nasal cannula) it is important to properly oxygenate the patient and maintain an acceptable blood oxygen saturation level. This may be particularly important for morbidly obese patients, who are more likely to quickly desaturate. In some cases it can also be useful to prevent a patient from being excessively oxygenated. A second consideration may be given to maintaining acceptable levels of carbon dioxide in the patient's blood and/or respiratory system. A third consideration may be given to reducing the gas flow rate used to deliver therapy if it can be shown that the gas flow rate used may be greater than the minimum necessary to maintain proper levels of oxygen saturation and carbon dioxide. A further consideration is to reduce the risk of barotrauma and/or gastric distension.

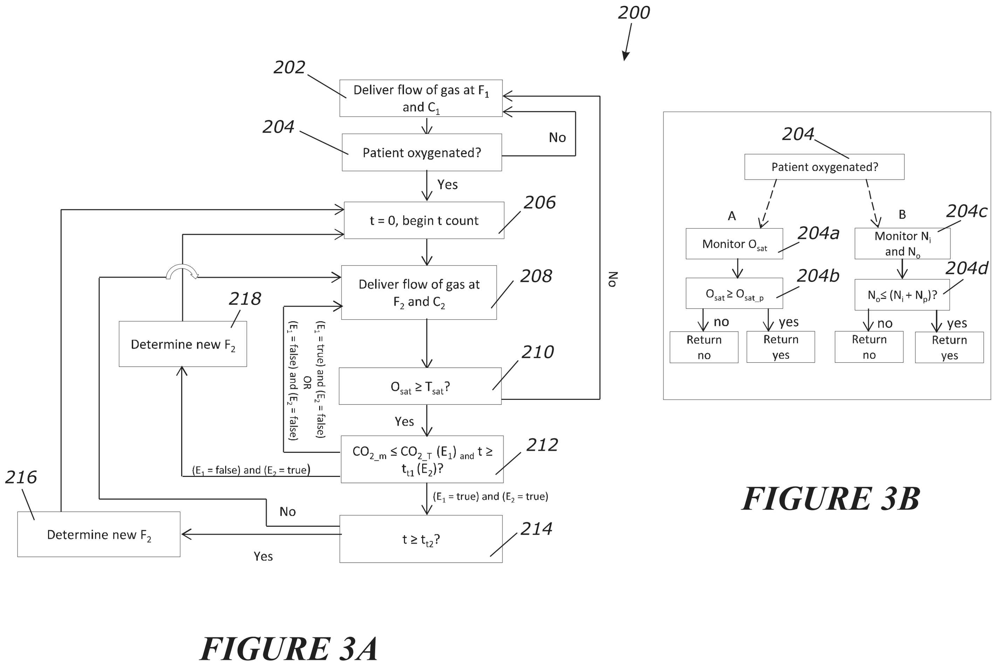

Accordingly, with reference to the non-limiting exemplary embodiment shown in Figures 3A-3B, at least parts of a respiratory therapy method 200 are shown. The methods of Figures 3A-3B may be implemented using the system of figure 1. The respiratory therapy method 200 may be practiced by, for example, the controller 108 as shown in Figure 1. Additionally, in some configurations not all steps are necessary or need to be practiced. In step 202, a patient can be oxygenated (using, for example, the respiratory therapy system 100) by delivering a first gas therapy T, wherein respiratory gases of a first composition $C_1$ at a first flow rate $F_1$ are delivered. The first gas therapy $T_1$ may a gas therapy primarily configured to oxygenate the patient. In some configurations, the patient may be spontaneously breathing at this stage. The first composition $C_1$ may comprise, for example, 100% oxygen, although in other configurations air/oxygen mixtures (for example, 50% oxygen and 50% air) may be used. The first flow rate may be a high flow therapy rate, and may be, for example, about 30 to about 100 LPM, or may be one of the other high flow therapy rates described above or elsewhere in this disclosure. Periodically (for example, after every x number of milliseconds, where x may be a predetermined value), a check may be made to ascertain if the patient is sufficiently oxygenated (see step 204). If the patient is determined to not be sufficiently oxygenated, step 202 repeats (e.g. the delivery of respiratory gases at $C_1$ and $F_1$ continues). Alternatively, the delivery of respiratory gases may occur at an increased flow rate. The composition of the gas may have more oxygen than the previously used gas. If the patient is determined to be sufficiently oxygenated, step 206 is called.

Step 204 comprises a check to ascertain if the patient is sufficiently oxygenated. This step may be practiced in a variety of ways, including but not limited to methods A and/or B as shown in Figure 3B. In method A, the patient's blood oxygen saturation $O_{sat}$ may be determined (see step 204a) (for example, using the physiological sensor module 121 described elsewhere with reference to Figure 1, or more specifically a pulse oximeter as described elsewhere in this disclosure with reference to the physiological sensor module 121). If the determined $O_{sat}$ is greater than or equal to a threshold blood oxygen saturation level $O_{sat\_p}$ (which may be a predetermined threshold blood oxygen saturation level $O_{sat\_p}$) (see step 204b), the patient may be deemed to have been sufficiently oxygenated and step 204 may return a 'yes.' Otherwise, if the measured $O_{sat}$ is less than the $O_{sat\_p}$, the patient may be deemed to not have been sufficiently oxygenated and step 204 may return a 'no.'

With further reference to step 204, in method B, the quantity of nitrogen gas inhaled by the patient ($N_i$) and the quantity of nitrogen gas exhaled by the patient ($N_o$) may be measured (for example, using the sensing module 120 described elsewhere with reference to Figure 1) (see step 204c). It will be appreciated that the method of determining oxygenation level based on nitrogen is subject to at least some nitrogen being delivered, such as about 1% nitrogen. In addition, it will be understood that this process is limited to situations where the patient is breathing spontaneously. During oxygenation, it is expected that $N_o$ will be greater than $N_i$ if the gas therapy delivered is continuing to further oxygenate the patient, as oxygen can be observed to displace nitrogen in the respiratory airways, further providing a buffer against hypoxemia. Furthermore, as the patient becomes more oxygenated, the $N_o$ would be expected to move closer to the $N_i$, or stated in another manner, the ratio of $N_o$:$N_i$ would be expected to approach 1 as the patient becomes fully oxygenated. As the $N_o$:$N_i$ ratio approaches 1, it generally takes more time to further increase the oxygenation of the patient - for example, it may take less time for the patient to go from 50%-55% oxygenated than for the patient to go from 90% to 95% oxygenated, and at some point it may not be efficient for a physician to continue to oxygenate the patient past a certain threshold. In step 204d, then, a check may be made to determine if the $N_o$ is less than or equal to the $N_i$ plus a nitrogen variance value $N_p$ (which may be about zero or which may be a positive value, and may be a predetermined value) signifying a tolerable deviation of $N_o$ from $N_i$ (e.g. $N_o \leq (N_i + N_p)$), or to determine if the ratio $N_o$:$N_i$ is less than or equal to a predetermined nitrogen ratio $N_r$ (which may be a predetermined nitrogen ratio $N_r$, and which may about 1 or about 1 plus a nitrogen ratio variance value $N_{p\_r}$ (which may again be a predetermined nitrogen ratio variance value $N_{p\_r}$) (e.g. $N_o$:$N_i \leq$ (about $1 + N_{p\_r}$)). If $N_o \leq (N_i + N_p)$ (or $N_o$:$N_i \leq$ (about $1 + N_{p\_r}$)), the patient may be deemed to have been sufficiently oxygenated and step 204 may return a 'yes.' Otherwise, if $N_o > (N_i + N_p)$ (or $N_o$:$N_i >$ (about $1 + N_{p\_r}$)), the patient may be deemed to not have been sufficiently oxygenated and step 204 may return a 'no.'

In some configurations, methods A and B of Figure 3B may be combined. For example, if the $O_{sat}$ is within a range of values for which it is not clear if the patient is or is not sufficiently oxygenated, the nitrogen values $N_i$ and $N_o$ may be monitored to confirm the result of the check. Similarly, if either the $N_o$ or the ratio $N_o/N_i$ are within a range of values for which it is not clear if the patient is or is not sufficiently oxygenated, the $O_{sat}$ may be monitored to confirm the result of the check. In some configurations, several iterations of one or more of methods A and/or B may be run consecutively for periods of time (e.g. predetermined periods of time) to verify the accuracy of the determination of whether or not a patient is sufficiently oxygenated. In some configurations, if methods A and B disagree, a third 'tiebreaker' determination may be performed based on, for example, inhaled and/or exhaled $CO_2$. Other tests or determinations or variations of the tests or determinations disclosed above or elsewhere in this disclosure may be contemplated by one skilled in the art upon study of this disclosure, and are hereby incorporated herein.

In an alternative configuration, the system 100 and method may determine a ratio of inhaled oxygen to exhaled oxygen to determine if there is a sufficient amount of oxygenation of the patient. In this alternative, the controller 108 is adapted to measure a first quantity of oxygen inhaled by the patient Oi and a second quantity of oxygen exhaled by the patient Oo. The controller 108 is configured to control the flow source 102 to deliver a first flow therapy, receive the measured Oi and Oo over at least one respiratory cycle, and control the flow source 102 to continue delivering the first flow therapy or to deliver a second flow therapy on the basis of a function of the measured Oi and Oo.

The concentration/amount of inhaled oxygen Oi will be known because it is set by the controller 108. For example, the system will deliver a maximum of 99% oxygen as inhaled oxygen. The expired oxygen Oo can be measured by a sensor in the physiological sensing module 121. The expired oxygen will increase and approach about 90% at which point the patient has been oxygenated to an acceptable level. A lower acceptable level may be about 80%. At the acceptable level, the expired oxygen may reach a plateau. At that stage, the ratio of inhaled oxygen Oi to exhaled oxygen Oo will approach 0.90. The patient will also expired some water and some $CO_2$. A lower acceptable level may be about 0.80. It will be understood that this process is limited to situations where the patient is breathing spontaneously.

With further reference to Figure 3A, in step 206 a time count variable t may be set to zero and may be set to track the passage of time. In step 208, as it has been determined in step 204 that the patient is sufficiently oxygenated, the patient can be treated with a second gas therapy $T_2$ wherein respiratory gases of a second composition $C_2$ at a second flow rate $F_2$ are delivered. The system 100 may be configured to deliver anaesthetic agents at this point, and/or inform or warn, for example, a physician, that pre-oxygenation is complete and optionally that the process of intubation or endoscopy may be started. In some configurations, the trigger for the delivery of anaesthetic agents and/or alarm of the physician may be the determination of the blood oxygen saturation level reaching or exceeding a threshold blood oxygen saturation level. In other configurations, the trigger for the delivery of anaesthetic agents and/or alarm of the physician may be the determination of the cessation of breathing. The alarm may be visual, oral, and/or tactile. For example, there may be a suitable user interface, such as a touch screen or coloured lights. An example may be red lights indicating that the patient should continue to be pre-oxygenated, orange indicating nearing adequate pre-oxygenation and green indicating that pre-oxygenation is complete.

In other configurations, the physician may be alarmed or informed that the intubation or endoscopy should be started based on some other sensed data (for example, data of various sensors described elsewhere in this disclosure) (for example, when data parameters meet or exceed predetermined or derived or calculated threshold values). In some configurations, and particularly if the patient is being deeply sedated with anaesthetic agents, the patient may not be spontaneously breathing. The second gas therapy $T_2$ may be a gas therapy primarily configured to maintain adequate ventilation of the patient and manage dead space, including but not limited to anatomical dead space. The second gas composition $C_2$ may comprise, for example, 80% air and 20% oxygen, although in other configurations other air/oxygen mixtures or just air (e.g. 100% air) or 100% oxygen may be used. In some configurations, the second gas composition $C_2$ may comprise less oxygen than the first gas composition $C_1$ (e.g. the first gas composition $C_1$ may comprise a first level of oxygen $G_{o1}$ which is greater than a second level of oxygen $G_{o2}$ of the second gas composition $C_2$). In some configurations, the second gas composition $C_2$ may comprise more oxygen than the first gas composition $C_1$ (e.g. the first gas composition $C_1$ may comprise a first level of oxygen $G_{o1}$ which is less than a second level of oxygen $G_{o2}$ of the second gas composition $C_2$). In some configurations, the second gas composition $C_2$ may comprise more air than the first gas composition $C_1$ (e.g the first gas composition $C_1$ may comprise a first level of ambient gas $G_{a1}$ which is less than a second level of ambient gas $G_{a2}$ of the second gas composition $C_2$). In some configurations, the ratio $G_{o1}:(G_{a1}+G_{o1})$ may be greater than the ratio $G_{o2}:(G_{a2}+G_{o2})$.

In step 210, a check may be made to see if the patient is adequately oxygenated. The blood oxygen saturation $O_{sat}$ may be determined (for example, using the physiological sensor module 121 described elsewhere with reference to Figure 1). If the $O_{sat}$ is greater than or equal to a threshold blood oxygen saturation $T_{sat}$ (which may be a predetermined threshold blood oxygen saturation $T_{sat}$), a 'yes' may be returned and step 212 may be performed. If the $O_{sat}$ is less than the $T_{sat}$, a 'no' may be returned and step 202 may be performed again to attempt to again oxygenate the patient to an acceptable level. Step 202 may occur at a flow rate that is higher than the previously used flow rate. The composition of the gas may have more oxygen than the previously used gas. In some configurations, one or more iterations of step 204 may be performed instead of step 210. In some such configurations, the $O_{sat\_p}$, the $N_p$ and/or the $N_{p\_r}$ may be set to different values if step 204 is practiced in place of step 210.

In step 212, a check may be made to see if the second gas therapy $T_2$ has been effective at managing the patient's blood carbon dioxide, ventilation, and/or dead space. Many tests or determinations for these variables may be utilized. In some cases it may take time before the effect of the second gas therapy $T_2$ on the above can be determined. In some configurations, a first Boolean value $E_1$ may be generated in response to determining if the patient's blood carbon dioxide $CO_{2\_m}$ (which may be found, for example, using the physiological sensor module 121 described elsewhere with reference to Figure 1) is less than or equal to a threshold blood carbon dioxide $CO_{2\_t}$ (which may be a predetermined threshold blood carbon dioxide $CO_{2\_t}$). $E_1$ may be set to 'true' if $CO_{2\_m}$ is less than or equal to $CO_{2\_t}$ (e.g. $E_1$ = true if $(CO_{2\_m} \leq CO_{2\_t})$) and may be set to 'false' if $CO_{2\_m}$ is greater than $CO_{2\_t}$ (e.g. $E_1$ = false if $(CO_{2\_m} > CO_{2\_t})$). Additionally, a second Boolean value $E_2$ may be generated in response to determining if the time count variable t set to zero in step 206 is greater than or equal to a first threshold time $t_{t1}$ (which may be a predetermined first threshold time $t_{t1}$). $E_2$ may be set to 'true' if t is greater than or equal to the first threshold time $t_{t1}$ (e.g. $E_2$ = true if $(t \geq t_{t1})$) and may be set to 'false' if t is less than the first threshold time $t_{t1}$ (e.g. $E_2$ = false if $(t < t_{t1})$). It should be understood that other variables may be measured in place of or in conjunction with the patient's blood carbon dioxide to determine the efficacy of the second gas therapy $T_2$, including but not limited to inhaled and/or exhaled $CO_2$.

If $(E_1 = false)$ and $(E_2 = false)$, it may be that the second gas therapy $T_2$ has not been delivered for long enough to achieve an acceptable $CO_{2\_m}$, and step 208 may be practiced again. If $(E_1 = true)$ and $(E_2 = false)$, the second gas therapy $T_2$ may have been delivered for long enough and/or at a high enough flow rate to achieve an acceptable $CO_{2\_m}$, but it may be useful to allow for additional time for the measured or determined $CO_{2\_m}$ to normalize or be verified after a number of measurements, and so step 208 may be practiced again, although in some configurations, the method 200 may practice step 214 instead. If $(E_1 = false)$ and $(E_2 = true)$, it may be that the flow rate $F_2$ delivered is too low or otherwise is not sufficiently effective. A new flow rate $F_2$ may be calculated in step 218. The new flow rate $F_2$ may be higher than the previously used flow rate $F_2$, and in some configurations the first threshold time $t_{t1}$ may be altered. The composition of the gas may have more oxygen than the previously used gas. The method 200 may then loop back to step 206. If $(E_1 = true)$ and $(E_2 = true)$ it may be determined that the delivered flow rate $F_2$ is sufficient and that enough time t has passed, and so step 214 may be practiced. Oxygen saturation can be used as a proxy measurement for blood CO2 since it is easier to measure O2 saturation.

In step 214, it may be found that the flow rate $F_2$ is higher than it needs to be. If a second threshold time $t_{t2}$ has been met or passed since the time count variable t was initialized in step 206 (e.g. if $t \geq t_{t2}$), a new flow rate $F_2$ may be calculated in step 216. The new flow rate $F_2$ may be less than the previously used flow rate $F_2$, and may be a percentage of the previously used flow rate or may be some other new flow rate $F_2$. If $(t < t_{t2})$, it may be determined that not enough time has passed to determine if the flow rate $F_2$ is higher than necessary, and step 208 may be practiced again.

As an alternative to steps 206 onwards, the flow may be set to a predetermined level eg: 30lpm, and maintained at that level until the patient is oxygenated (according to step 204) and then user is notified that the patient is oxygenated. In this alternative, there is no change in the flow rate, no change in the composition of the gas, and no time monitoring.

The flow rate may be increased if sufficient oxygenation is not occurring within the expected time frame, for example 3 or 5 minutes. In this case the monitoring after apnea would not proceed (ie: steps 206 onwards).

Flow may be increased by user once the patient is asleep or at the onset of apnea, for example to 70lpm. Or the clinician may notify the system that patient is asleep or an increase in flow is required and the system automatically increases the flow to 70lpm, for example.

In addition, other systems and/or kits may be useful for delivering flow therapy (e.g. high flow therapy) to patients undergoing, for example, intubations or upper endoscopies. Figure 4 shows a respiratory therapy system 100. The respiratory system 100 comprises a respiratory therapy apparatus. The respiratory therapy apparatus may include a flow source, a humidifier, and/or an integrated flow source/humidifier apparatus. A conduit 112 is connected to the respiratory therapy apparatus, and the conduit 112 can extend from the respiratory therapy apparatus to a patient interface, which may be a nasal cannula 116 as similarly described with reference to Figure 1 elsewhere in this disclosure. The nasal cannula 116 is configured to be detachably connected to the conduit 112. The conduit 112 is integrally moulded or be in the form of a single part or continuous piece with the respiratory therapy apparatus, or may be permanently attached to the respiratory therapy apparatus (although in other configurations the conduit 112 and the respiratory therapy apparatus may be separable) and the nasal cannula 116 may be the only part of the system 100 that is intended to be disposable. In some configurations, the respiratory therapy apparatus 143, the conduit 112, and the nasal cannula 116 may all be integrally moulded or in the form of a single part, or in some configurations the respiratory therapy apparatus may be permanently connected to one side of the conduit 112 and the nasal cannula 116 may be permanently connected to another side of the conduit 112.

The patient-facing outlet 140 of the conduit 112 may connect to the gas inlet 142 of the nasal cannula 116 through a variety of attachment mechanisms known to those skilled in the art, including but not limited to latch/catch arrangements or bayonet fittings.

The system 100 comprises a disinfection arrangement 135 adapted to disinfect at least a section of the conduit 112, although in some configurations the disinfection arrangement 135 may not be present. The disinfection arrangement 135 may be, for example, attached to the conduit 112. The disinfection arrangement 135 may comprise, for example, one or more electromagnetic radiation emitters (positioned inside the conduit 112, for example). The one or more emitters may comprise an ultraviolet light source (e.g. UV LED), a microwave emitter, and/or some other radiation emitter configured to sterilize at least a portion of the gas flow path inside the conduit 112. In some configurations, the system 100 can be controlled to channel high temperature gases through the conduit 112 to assist in disinfection. The high temperature gases may be configured to carry enough moisture to increase the total enthalpy of the gases, further promoting disinfection of the conduit 112. In some configurations, the system 100 can be controlled such that a conduit heater of the conduit 112 is activated to assist in disinfection. Means for sterilizing the gas flow path can reduce concerns of patient infection through the introduction of undesired pathogens.

As further shown in Figure 4, the respiratory therapy apparatus 143 may comprise a controller 108. The controller 108 may be in electronic communication with the disinfection arrangement 135 and may be adapted to control the on/off state and/or intensity of disinfection of at least a part of the disinfection arrangement 135. The controller 108 may control the disinfection arrangement 135 dependent on whether or not the nasal cannula 116 is attached to the conduit 112 or on whether or not the nasal cannula 116 is being worn by the patient. If the nasal cannula 116 is removed from the conduit 112, and particularly if the nasal cannula 116 is removed from the conduit after usage of the nasal cannula 116 by a patient, or if the nasal cannula 116 is at first worn on a patient's face and then removed, the controller 108 may be configured to activate the disinfection arrangement 135 and/or change the operation parameters of the disinfection arrangement 135. In some configurations, a sensor module 144 may be used to determine if the nasal cannula 116 is connected to the conduit 112 and/or if the nasal cannula 116 is fitted to or being used by the patient. The sensor module 144 may comprise, for example, a pressure sensor and/or a flow sensor. Signals outputted by the sensor module 144 and/or values derived therefrom may be used by the controller 108 to assist in the control of the disinfection arrangement 135. (e.g. via determining the presence of absence of the nasal cannula 116 (including the presence or absence of a sensor or an electrical component of the nasal cannula 116) or if the nasal cannula 116 is being worn by the patient, for example).

A one-way valve 138 is shown in Figure 4 as lying in the conduit 112 near the patient-facing outlet 140, although in some configurations the one-way valve 138 may be placed elsewhere (such as but not limited to another position in the conduit 112 or in the nasal cannula 116). The one-way valve 138 may be configured to prevent the flow of exhaled gases through the conduit 112. The one-way valve 138 may help to protect the sterility of the conduit 112 and/or other parts or components of the respiratory therapy apparatus 143 and/or system 100.

In some cases, then, the systems, apparatus, kits and methods shown or implied in the disclosure with reference to Figure 4 may be beneficially used to treat a plurality of patients using substantially the same equipment, except in some cases for the nasal cannula 116 which may be disposable.

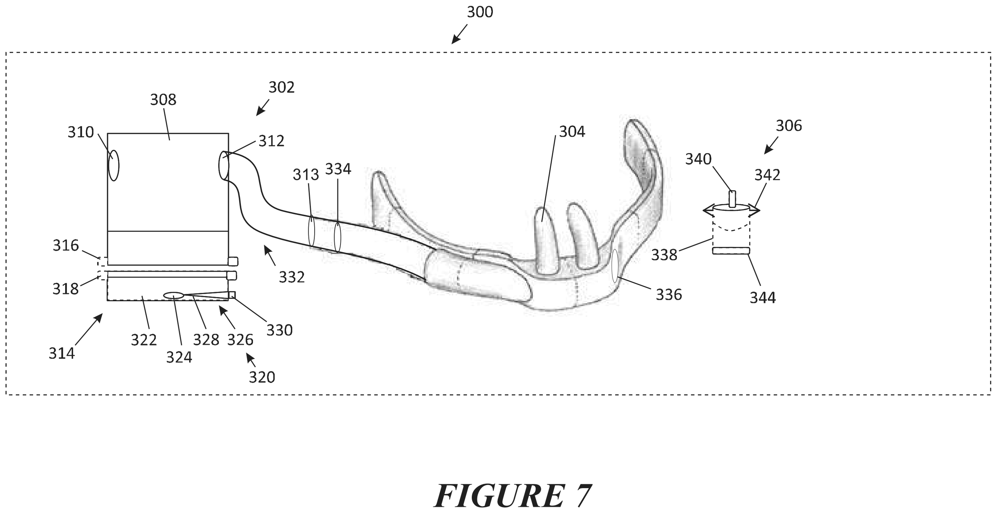

In other cases, it may be that a medical professional treating the patient may wish to use a partially or fully disposable kit to oxygenate and/or treat the patient with flow therapy (e.g. high flow therapy). In Figure 7, a respiratory therapy kit 300 is shown. The respiratory therapy kit 300 comprises a humidifier 302 and a patient interface (which is shown as a nasal cannula 304, but which may include other interfaces such as but not limited to those listed elsewhere in this disclosure). The humidifier 302 comprises a reservoir 308. The reservoir 308 comprises an inlet 310 that may in use be connected to a flow source and an outlet 312 that may be connected to the nasal cannula 304. As shown the outlet 312 may be pre-connected to nasal cannula 304 through a conduit 332. In some configurations, the conduit 332 may be integrally moulded or be in the form of a single part with the humidifier 302 and the nasal cannula 304, and/or the conduit 332 may be permanently connected on one side to the humidifier 302 and on another side to the nasal cannula 304. The reservoir 308 may be pre-filled with a quantity of water. The quantity of water may be small, e.g. in the range of about 20 to about 40 milliliters of water, or may be up to 250mL.

The respiratory therapy kit 300 may comprise a heating device 314. The heating device 314 may be adapted to heat water in the reservoir 308. The heating device 314 may be non-permanently attachable to the reservoir 308, may be permanently attached to the reservoir 308 or may be integrally moulded with at least a portion of a wall of the reservoir 308. The reservoir 308 may comprise, for example, a latch 316 that may be joined with a catch 318 attached to the heating device 314, or vice versa. The heating device 314 may comprise a chemical heater. The chemical heater may comprise, for example, a reservoir 322 configured to contain a quantity of a reactive reagent liquid such as but not limited to water and a bubble or payload 324 comprising a quantity of a reactive reagent solid such as but not limited to magnesium metal encased by a layer or membrane adapted to isolate the reactive reagent solid from the reactive reagent liquid. The heating device 314 may comprise a manually actuatable switch 320 adapted to allow for a user to activate the heating device 314. For example, the manually actuatable switch 320 may comprise a rod 326 comprising a sharp end 328 adapted to pierce the membrane of the payload 324 and a button 330. Pressing on the button 330 may force the sharp end 328 to thrust towards the payload 324 and pierce the membrane, exposing the reactive reagent solid to the reactive reagent liquid and causing an exothermic chemical reaction that may heat water in the reservoir 322. The button 330 may be biased with a spring or other biasing mechanism to prevent accidental and/or premature piercing of the membrane of the payload 324.

The use of a relatively small volume of water in the reservoir 308 promotes a relatively quick heat up and dispersal of moisture into gases passing through the humidifier 302.

With further reference to Figure 7, the nasal cannula 304 may further comprise a port 336. As shown the port 336 can be on a side of the nasal cannula 304 opposite to the side through which respiratory gases are delivered, although the port 336 may be placed elsewhere on the nasal cannula 304. The port 336 may be configured to accept a drug delivery device. The respiratory therapy kit 300 may also comprise a drug delivery device 306 adapted to be located in the port 336. The drug delivery device 306 may be detachably connectable to the port 336 through, for example, a latch 342 that may fit in a catch in or on the nasal cannula 304 at or near the port 336. The drug delivery device 306 may comprise a spigot or head 340 adapted to deliver, aerosolize or nebulize medication stored in a pre-filled medication reservoir 338. The medication reservoir 338 may comprise, for example, a quantity of lidocaine or another local anaesthetic agent. The drug delivery device 306 may also comprise a manually actuatable metering mechanism 344 adapted to allow for the release of medication. For example, the metering mechanism 344 may comprise a piston that may be pushed by a user to propel medication in the medication reservoir 338 through the spigot 340. The piston may be biased with a spring or other biasing mechanism to prevent premature dispensation of the medication in the medication reservoir 338.

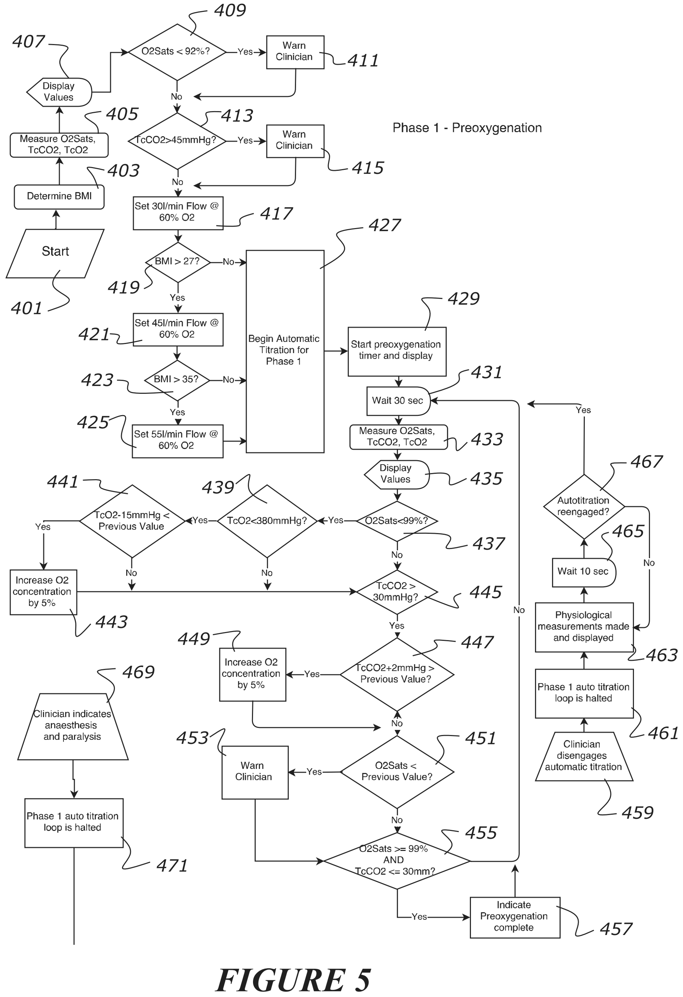

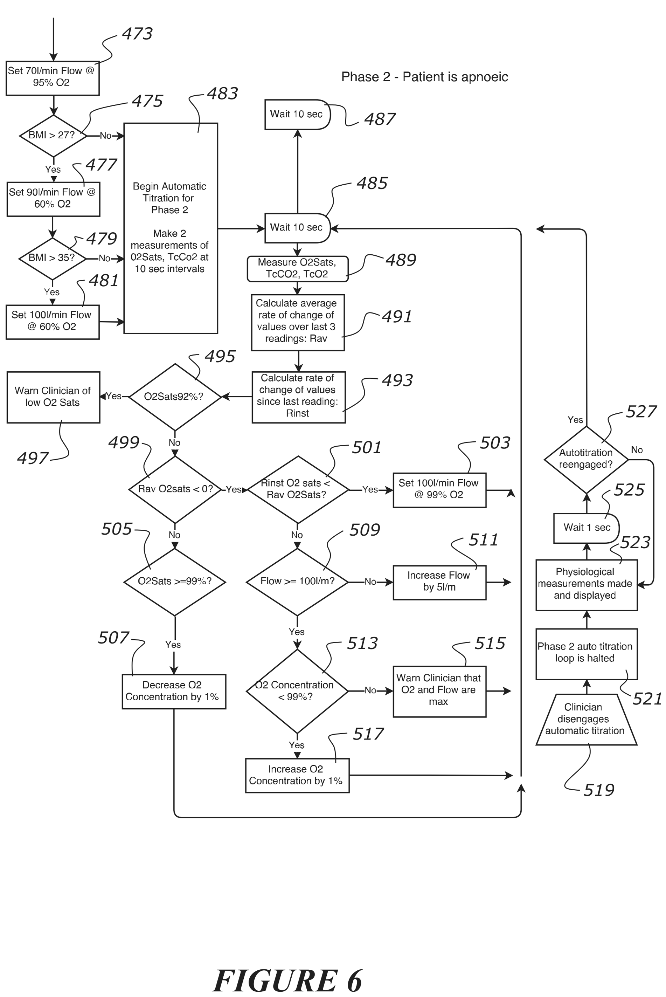

With reference to Figures 5 and 6, an example of a respiratory therapy method is shown. The method may be performed using the system shown and described in relation to figures 1 and 2. Figure 5 shows phase 1: the preoxygenation phase where patient is breathing. Figures 5 and 6 and the associated description refer to the term "titration". It will be understood that titration refers to controlling the flow rate, concentration of gases supplied, and controlling other parameters of the flow of gases supplied to the patient. The methods of figures 5 and 6 are executed by the controller such that the system can automatically control flow based on the measured parameters. The method is an automated approach to titration that is executed/implemented by the system of figure 1.

The method described in relation to figures 5 and 6 aims to:

- achieve optimal oxygenation of the patient as rapidly as possible during the preoxygenation phase when the patient is breathing

- after saturation, maintain blood oxygen saturation levels as high as possible and warn the clinician if the oxygen saturation level falls below a threshold level.

After saturation, also maintain blood $CO_2$ levels as low as possible, and warn the clinician if the levels rise above a threshold level.

The physiological parameters of the patient are monitored by the physiological sensor module 121. The specific signals used from the physiological sensor module 121 in this method are the partial pressure of oxygen and the partial pressure of $CO_2$ in the patient (both are measured by the transcutaneous monitor), and the blood oxygen saturation.

It will be understood that the method described in relation to figures 5 and 6 is an example only. Alterations may be made to the method. For example, alternative methods could use additional signals from the physiological sensor module 121 in addition to, or instead of, the signals in this example method.

The figures shown and referred to in relation to figures 5 and 6, such as the concentrations of oxygen in the gas supplied to the cannula, threshold values of BMI, transcutaneous O2 and Transcutaneous $CO_2$ readings are by way of example only. Other values may be used and may be determined by a clinician.

The method starts at 401. In step 403, the patient's BMI is determined.

In step 405, the blood oxygen saturation level is measured by the physiological sensor module 121. In step 409, if the blood oxygen saturation level is below 92% on room air, the system provides a warning to the physician in step 411. This might be an audible warning, or a warning displayed on the display screen of the controllerinput/output module 110.

In step 405, the transcutaneous $CO_2$ reading is also measured by the physiological sensor module 121. If this is greater than 45mmHg (step 413), the module provides a warning to the physician in step 415. This might be an audible warning, or a warning displayed on the display screen of the controllerinput/output module 110.

The transcutaneous O2 reading is measured by the physiological sensor module 121.

In step 407, the values are displayed.

For patients of BMI<27, an initial humidified flow of 60% oxygen, 40% nitrogen is supplied through the cannula at 30l/min (step 417). This corresponds to a mix of 50% oxygen, 50% air.

For patients of BMI between 27 and 35 (step 419), an initial humidified flow 60% oxygen, 40% nitrogen is supplied through the cannula at 45l/min(step 421). This corresponds to a mix of 50% oxygen, 50% air.

In step 423, for patients of BMI above 35, an initial humidified flow of 60% oxygen, 40% nitrogen is supplied through the cannula at 55l/min (step 425). This corresponds to a mix of 50% oxygen, 50% air.

In step 427, automatic titration for phase 1 is started and the preoxygenation timer is started to monitor the preoxygenation time (step 429). The time is displayed by the controller input/output module 110 at 1 sec intervals.

The At step 429, the blood oxygen saturation, transcutaneous O2, and Transcutaneous $CO_2$ of the patient are measured and displayed at 30 second intervals by the physiological sensor module (step 429).

At each 30 second interval (step 431) measurements of blood oxygen saturation level, transcutaneous O2, and Transcutaneous CO2 are made by the physiological sensor module 121 at step 433, displayed at step 435, and stored in the controller 108.

In step 437, if the oxygen saturation level is lower than 99%(437),% and if the transcutaneous O2 level is lower than 380mmHg (in step 439) then the measured transcutaneous O2 level is compared with the measurement made 30 sec earlier in step 441. If the new transcutaneous O2 level is less than the old transcutaneous O2 level plus 15mmHg, the oxygen content in the gas composition is increased at step 443. For example, if the initial transcutaneous O2 reading was 155mmHg and the new reading after 30 seconds was less than 160mmHg, the oxygen content in the gas supplied to the cannula might be increased by 5% to 65% and the nitrogen content would then be consequently decreased to 35% - this corresponds to a gas mix of 43.75% air and 56.25% oxygen.

In step 445, if transcutaneous O2 is equal or greater than 380mmHg, the oxygen concentration in the flow to the cannula is not adjusted.

In step 437, if the oxygen saturation is equal to or greater than 99%, the oxygen concentration in the flow to the cannula is not adjusted.

In step 445, if the transcutaneous CO2 level is greater than 30mmHg, then, the measured transcutaneous CO2 level is compared with the measurement made 30 sec earlier (in step 447). If the new transcutaneous CO2 level is greater than the old saturation level minus 2mmHg, the oxygen content in the gas composition is increased (in step 449). For example, if the initial transcutaneous CO2 reading was 40mmHg and the new reading after 30 seconds was greater than 38mmHg, the oxygen content in the gas supplied to the cannula might be increased by 5% to 65% and the nitrogen content would then be consequently decreased to 35% - this corresponds to a gas mix of 43.75% air and 56.25% oxygen.

In step 445, if the transcutaneous O2 level is less than or equal to 30mmHg, the oxygen concentration in the flow to the cannula is not adjusted.

In step 451, the measured blood oxygen saturation level is compared with the blood oxygen saturation level measured 30 sec earlier.

If the blood oxygen saturation level has fallen, then a warning is given (in step 453). This might be an audible warning or a warning displayed on the screen of the physiological sensor module 121. If the blood oxygen saturation level has not fallen, then a warning is not given (step 453).

If the blood oxygen saturation is greater than or equal to 99% AND the transcutaneous CO2 reading is less than or equal to 30mmHg( at step 455), then, at step 457 a message to indicate the end of the pre-oxygenation phase is displayed on the display of the physiological sensor module (457).

If those conditions are not met, a new set of measurements is taken by the physiological sensor module 121 after a 30 sec interval (in step 431), and the automatic titration method for preoxygenation continues (in step 433).

At any point during the Phase 1 method, the clinician may disengage automatic control of the oxygen concentration and flow rate applied to the cannula and then manually adjust the concentration of oxygen in the flow to the cannula and the flow rate while observing the physiological measurements. During this time, the rate of measurement and display of physiological parameters increases to once every 10 seconds. When this adjustment has been made, the clinician has the option of re-engaging automatic control of these quantities. The method then restarts from the beginning of section j.

When the display of the controller input/output module 110 indicates that preoxygenation is complete, the clinician may move on to anaesthetise and paralyse the patient.

### Phase 2 (Patient is apnoeic)

When the patient has lost consciousness and paralysis has taken effect, the clinician indicates this to the controller (108 in step 469), for example, by pressing a button or other means (in step 471). The controller 108 now stops executing the part of the method for Phase 1 of the procedure and enters the method for Phase 2. It sets initial conditions for Phase 2 (step 473).

For patients of BMI<27(step 475), an initial humidified flow of 95% oxygen, 5% nitrogen is supplied through the cannula at 70l/min.

For patients of BMI between 27 and 35 (step 479), an initial humidified flow 95% oxygen, 5% nitrogen the supplied through the cannula at 80l/min.

For patients of BMI above 35 (step 479), an initial humidified flow of 95% oxygen, 5% nitrogen is supplied through the cannula at 90l/min.

The physiological sensor makes initial measurements (for Phase 2) of the oxygen saturation level of the blood, the transcutaneous O2, and the transcutaneous CO2 and these values are stored in the controller (in step 483).

Throughout Phase 2 of the procedure, the physiological sensor module 121 makes measurements of the oxygen saturation level of the blood, the transcutaneous O2, and the transcutaneous CO2 (other parameters may also be measured) at 10 second intervals (step 489). The values are displayed and stored in the controller 108.

When each new set of measurements is made, the controller 108 calculates the average rate of change of the measured parameters over the past three readings (step 491), and the rate of change of the parameters since the last reading (step 493). These values are used together to determine if the rate of change of each parameter is increasing or decreasing with time.

At each 10 second interval (step 485) if the blood oxygen saturation level is less than 92% (in step 495), then a warning is given to the clinician (in 497). This may an audible warning, or indicated on the controller, the display of the input/output module 110, or both.

If the blood oxygen saturation level is not less than 92% (in step 495), then a warning is not given to the clinician.

If the average rate of change of blood oxygen saturation is negative (in step 499), and if the rate of change of blood oxygen saturation is increasing (in step 501), then the flow is increased to 100l/min and the concentration of oxygen in the flow is increased to 99%, with the remaining 1% of gas supplied being nitrogen (in step 503).

If the rate of change of blood oxygen saturation level is not increasing, and if the flow is less than 100l/min (in step 509), then, the flow is increased by 5l/min (in step 511).

If the flow is 100l/min, and if the oxygen concentration in the flow is less than 99% (step 513), then the oxygen concentration is increased by 1% (in step 517) with a consequent decrease in the nitrogen concentration.

If the oxygen concentration is 99% (in step 513), a warning is given to the clinician that the patient it not responding to increases in oxygen concentration and flow (in step 515). This warning may be audible, or it may be displayed on the display of the controller, or it may be both.

If the average rate of change of blood oxygen saturation is zero or positive (in step 499) and if the average level of blood oxygen saturation is 99% or greater (in step 505), then the concentration of oxygen in the flow is reduced by 1% (in step 507) with a consequent increase in the concentration of nitrogen. If the average level of blood oxygen saturation is less than 99% (in step 505), then no change is made to the concentration of oxygen in the flow.

At any time during the method from step 485 onwards, the clinician may disengage automatic control of the gas composition and flow supplied to the cannula and make manual adjustments (steps 519 and 521). Under these circumstances, the rate of measurement and display of physiological parameters (in step 523) increases to once per second (in step 525). The clinician is then able to re-engage automatic control of the gas composition and flow to the cannula (in step 527). The Phase 2 method then restarts at step 485.

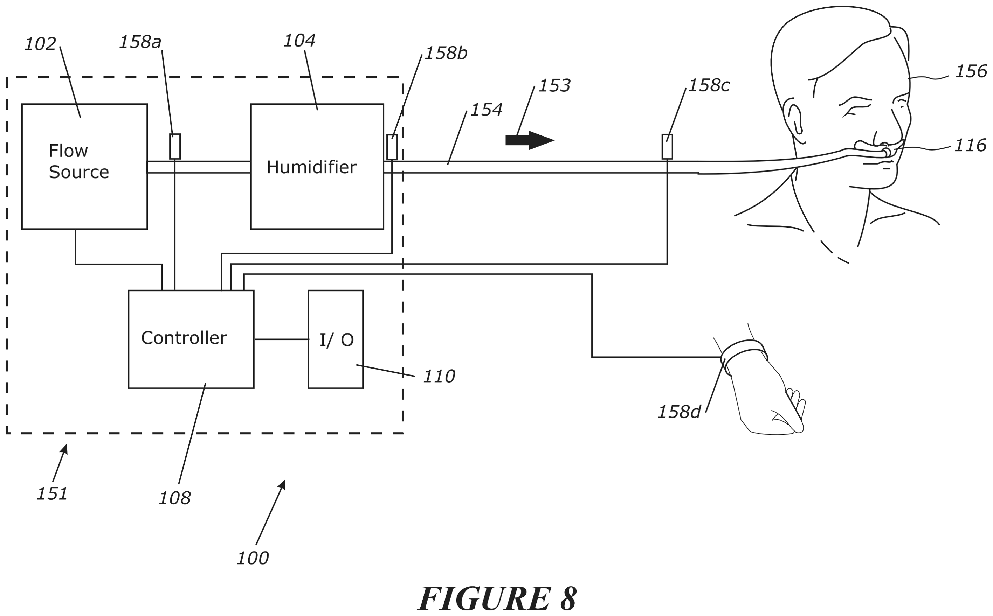

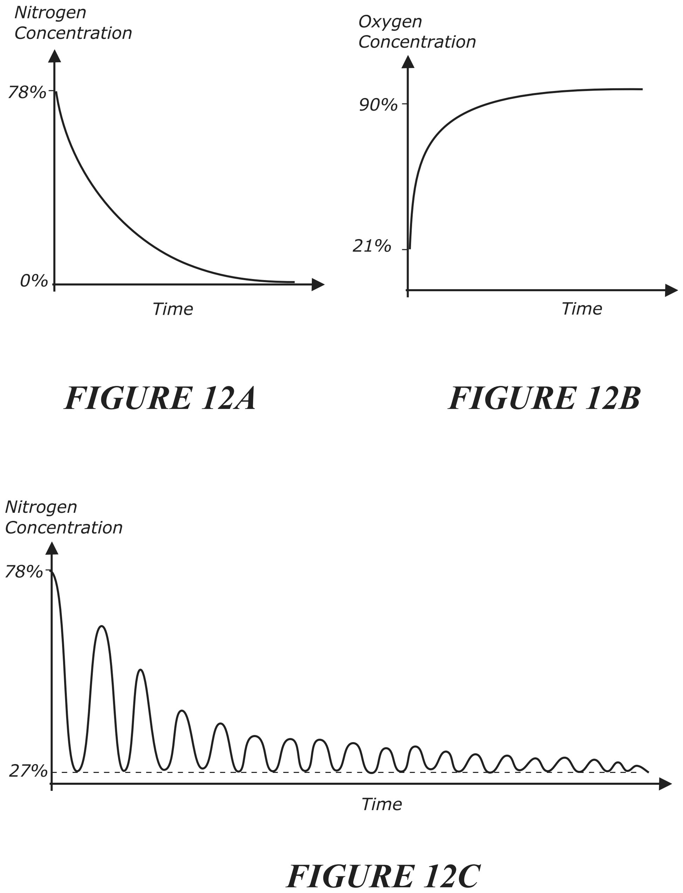

With reference to figures 12a to 12c, in some configurations, the present methods, systems and/or apparatus may be used to estimate or determine the remaining time until the patient has been sufficiently pre-oxygenated. The graphical outputs of figures 12A to 12C may be used by the controller of figure 1 or figure 8 to determine sufficient oxygenation of the lungs.

Additionally or alternatively, the present methods, systems, and/or apparatus may be used to determine the likelihood of the pre-oxygenation procedure achieving a target end-tidal oxygen level for the patient. Additionally or alternatively, the present methods, systems and/or apparatus may be used to determine and/or monitor the end-tidal oxygen level of a patient undergoing the pre-oxygenation procedure. Additionally or alternatively, the present methods, systems and/or apparatus may be used to determine if a patient has been sufficiently pre-oxygenated during the pre-oxygenation procedure. Additionally or alternatively, the present methods, systems and/or apparatus may be used to determine and/or monitor changes in the pre-oxygenation level of a patient undergoing the pre-oxygenation procedure.

The present method may involve measuring and/or tracking the concentration of one or more respiratory gases in the patient's expired gas. Pre-oxygenation can also be thought of as de-nitrogenation, as it is the nitrogen within the lungs that is being displaced by a high inspired oxygen concentration. End-tidal oxygen and/or nitrogen can therefore be measured to give an indication on the progression of pre-oxygenation.

The gas analysers may be used to measure the concentration of the respiratory gas(es) to be monitored. Specific gas analyser, for example, an oxygen gas analyser or a nitrogen gas analyser may be used to track the end-tidal concentration of the relevant gas.

Mass spectrometry may be used to measure the fractional concentrations of the different gases in the patient's expired gas (which is collected for analysis). One or more of these time-varying concentrations may be monitored.

Measurement and/or tracking of the concentration of the one or more respiratory gas may be performed substantially in real time.

Continuous gas measurements could also be used. For example, oxygen and nitrogen could be continuously measured during breathing throughout the pre-oxygenation procedure. Figure 12C shows an example of a possible trace of nitrogen. The offset of the inspiratory nitrogen concentration could be indicative of air entrainment and used to predict what the end-tidal oxygen value will be. Additionally, stabilisation of the waveform could be indicative of pre-oxygenation completion (as discussed in more detail below).

Additionally or alternatively, the present method involves measuring and/or tracking the concentration of one or more respiratory gases in the patient's blood (i.e., the blood saturation of the one or more gas). For example, the oxygen saturation of the patient's blood may be measured via pulse oximetry. Other methods of measuring the concentration of oxygen or other gases in the patient's blood may be utilised.

Sufficient pre-oxygenation is indicated by and/or corresponds to a stable or sustained (i.e., steady-state, plateau phase) end-tidal oxygen level and/or blood oxygen saturation level greater than 70%. In some configurations, sufficient pre-oxygenation is indicated by and/or corresponds to a sustained end-tidal oxygen level and/or blood oxygen saturation level greater than 90%. An example is shown in Figure 1B.

Sufficient pre-oxygenation is indicated by and/or corresponds to a stable or sustained (i.e., steady-state, plateau phase) end-tidal nitrogen level that is minimal or close to zero, as nitrogen is replaced with oxygen. An example is shown in Figure 12A.

Additionally or alternatively, the present method involves measuring and/or tracking the concentration of a tracer introduced to the patient's respiratory tract.

A tracer may be introduced into the lungs and the time to wash it out could be determined to monitor the pre-oxygenation level. Measuring and/or monitoring the concentration of the tracer in the patient's expired gas could be used to indicate when pre-oxygenation is complete. This will generally be at the point where there is substantially no tracer left.

Accordingly, in some configurations, sufficient pre-oxygenation is indicated by and/or corresponds to a stable or sustained (i.e., steady-state, plateau phase) end-tidal tracer level that is minimal or close to zero.

Tracers need to be highly insoluble in water (so they are not taken into the bloodstream) and readily diffusible (so they can be mixed with the air in the lungs). Examples of tracers which may be used include nitrogene, helium, argon and sulfur-hexafluoride, or any suitable combination thereof.

The tracer may be inhaled by the patient before the pre-oxygenation procedure is initiated.

The tracer may be inhaled by the patient during an initial warm-up phase of the pre-oxygenation therapy.

The concentration of the tracer in the patient's expired gas may be measured and/or monitored using, for example, an emission spectrometer, an ultrasonic gas analyser, or a respiratory mass spectrometer.

Additionally or alternatively, the present method involves measuring and/or tracking one or more physical properties of the patient's expired gas. Since pre-oxygenation results in changes to the gas mixture (e.g., decrease in nitrogen, increase in oxygen), the composition of the expired gas mixture may be correlated with the level of pre-oxygenation.

Physical properties of the gas mixture (e.g. density, viscosity, acoustic response etc.) could be measured to determine when the gas mixture has changed to the desired composition (e.g., from mainly nitrogen to mainly oxygen). Accordingly, such measurements may be used to estimate and/or monitor the fractional concentrations of the different respiratory gases in the patient's expired gas.

The concentration measurement(s) obtained via the methods described above over time may be displayed to the user, thus allowing the user to make an informed decision on the patient's pre-oxygenation state.

The concentration measurement(s) over time may be monitored, and when the measurement(s) has/have been in plateau for a certain amount of time (i.e., the measurement(s) reaches steady-state), this may indicate successful pre-oxygenation.

Further, if inspired oxygen is measured and is lower than what is being delivered from the source (due to air entrainment), the difference could be used as an indication of what the likely end-tidal oxygen value will be.

Further, instead of, or in addition to continuously monitoring the concentration measurement(s), measurement(s) obtained at or around the initial phase of the pre-oxygenation procedure may be used to predict the time remaining until sufficient pre-oxygenation.

Accordingly, in some configurations, the measurement(s) of the expired concentration and/or blood saturation of the one or more respiratory gases and/or tracer obtained at the initial phase of the pre-oxygenation procedure may be used to estimate the rate of change of these parameters. The rate of change may be fitted to a non-linear mathematical model. The time constant for the model may be calculated and multiplied with a treatment factor to estimate the time until sufficient pre-oxygenation may be achieved.

In one example, the change in oxygen concentration can be described by an exponential wash-in curve as it displaces nitrogen in the lungs. Conversely the change in nitrogen concentration can be described by an exponential wash-out curve as nitrogen is displaced from the lungs. These can be seen in Figures 12A and 12B. In other embodiments, the change in levels of other substances (e.g., carbon dioxide, a tracer, other introduced gases, etc.), may similarly be fitted to an exponential curve.

Once a suitable model is determined, the prediction methodology may be implemented in different ways, two of which will be described in more detail below.

In a first exemplary method, the interface supplying the pre-oxygenation flow to the patient is applied and pre-oxygenation therapy is initiated. The relevant expired gas level (i.e., one or more of oxygen, nitrogen, tracer, etc., as described above) is measured from the patient's first breath, and used as the baseline value for subsequent calculations. The method may be modified to incorporate one or more suitable methods for determining the patient's breath phase.

Using the first expired gas measurement as the baseline value, the time constant can be obtained. In the case of a wash-out curve (e.g., nitrogen, tracer, etc.), the time constant is the time taken for the measurement to drop to approximately 37% of its baseline value. For the oxygen wash-in curve, the time constant is the time taken for the measurement to increase by approximately 63% of the difference between the desired level and the level of the first expired gas measurement. It should be understood that these constants (and any other constants listed as examples in this specification) are approximations and may be varied according to the accuracy required. For example, a value between the range of 33% to 41% may be used for wash-out and a value of 59% to 67% may be used for wash-in.

The time constant may be multiplied by a treatment factor to obtain an estimate of when the wash-out or wash-in process is approximately 98% complete. In the example where an exponential curve is used to model the change in the expired gas level, a suitable treatment factor is four. Accordingly, this calculation could be used as the prediction for the remaining time left to pre-oxygenate the patient (i.e., an indication of the time until sufficient pre-oxygenation may be achieved).

There may be an optional step to multiply the 98% complete time with a safety factor. The safety factor may, for example, be an additional 20% to 50% of the estimated time.

The measurement(s) may be stopped once the level has dropped to approximately 37% of its baseline value (for wash-out models) or increased by approximately 63% of its baseline value (for wash-in models), since the model may be sufficient for predicting subsequent changes to the relevant gas levels.

Alternatively, the relevant expired gas concentration may be monitored throughout the pre-oxygenation process and the predicted time may be adjusted as the process continues. At the 98% complete time, the monitored value(s) may be assessed to see if the value(s) has/have stabilised and is pre-oxygenation is complete.

In a second exemplary method, the time constant may be estimated by dividing the patient's functional residual capacity by the patient's alveolar ventilation.

The patient's functional residual capacity may be estimated using several methods, including nitrogen washout tests, helium dilution, body plethysmography, and/or using a look up table (which may, for example, estimate that the functional residual capacity of a healthy patient is approximately 30 mL/kg and the functional residual capacity of an obese patient is approximately 15 mL/kg).

In one embodiment, the estimated value may be calculated by or input into a control interface of the pre-oxygenation therapy system or apparatus. Further, if the patient's weight is required to estimate the functional residual capacity, his/her weight may be input into the control interface or otherwise obtained (e.g., via weighing scales built into the hospital bed).

The patient's alveolar ventilation may be estimated using the following formula:

• Alveolar Ventilation = (Tidal Volume $\times$ Respiratory Rate) - (Dead Space Volume $\times$ Respiratory Rate)

The patient's tidal volume may be measured and/or monitored using any suitable method, e.g. spirometry, etc.

The patient's respiratory rate may be measured and/or monitored using any suitable method, e.g. pheumography, using a stethoscope, etc.

The patient's dead space volume may be estimated using several methods including nitrogen washout tests, measuring expired $CO_2$, estimating using the Bohr equation, or using a look up table (which may, for example, estimate that the dead space volume of a healthy patient is approximately 2 mL/kg).

The time constant is estimated as the quotient of the patient's functional residual capacity divided by the patient's alveolar ventilation. As with the first exemplary prediction method, the time constant may be multiplied by a treatment factor (e.g., four for an exponential model) to obtain an estimate of when the wash-out or wash-in process is 98% complete. Accordingly, this calculation could be used as the prediction for the remaining time left to pre-oxygenate the patient (i.e., an indication of the time until sufficient pre-oxygenation may be achieved).

Similarly, there is an optional step to multiply the 98% complete time with a safety factor.

Additionally, there is also an optional step to monitor one or more expired gas concentration throughout the process and adjust the pre-oxygenation time as the process continues. At the 98% complete time, the monitored value(s) may be assessed to see if the value(s) has/have stabilised and is pre-oxygenation is complete.

It should be noted, although the wash-in and wash-out process may generally be modelled as exponential processes, this is only one example. Under different respiratory support methods (CPAP, high flow, etc.) the process may be more accurately modelled using a different nonlinear function. Subsequently, the estimation of the time constant, and treatment of the time constant could be different depending on the treatment method.

The present disclosure also relates to suitable apparatus and systems for implementing the methods described. Specifically, the apparatus/system may comprises at least one or more sensors to measure and/or track one or more of the following: the concentration of one or more respiratory gases in the patient's expired gas, the concentration of one or more respiratory gases in the patient's blood (i.e., blood saturation), the concentration of a tracer introduced to the patient's respiratory tract, or one or more physical properties of the patient's expired gas.

The apparatus/system may further comprise a processor configured to fit the rate of change of the patient's expired concentration and/or blood saturation of said one or more respiratory gases and/or tracer to a non-linear model, determine a time constant for said model, multiply the time constant with a treatment factor to estimate the time until sufficient pre-oxygenation may be achieved, and indicate when the patient has been sufficiently pre-oxygenated.

The processor may be integral with or connected to the control interface of the pre-oxygenation therapy system, to obtain input from the control interface and/or to control the control interface.

The processor may further comprise a countdown timer configured to indicate to the medical professional the estimated time remaining until the patient is sufficiently pre-oxygenated. The timer may have a suitable user interface, for example in the form of coloured lights (e.g., red indicates that the patient should continue to be pre-oxygenated, orange indicates nearing adequate pre-oxygenation and green indicates that pre-oxygenation is complete). There could also be an audial (e.g., audio) or other visual indication when pre-oxygenation is complete. Haptic feedback may also be provided, optionally in combination with other types of indicators.

Pre-oxygenation may be delivered via high flow therapy. Further, pre-oxygenation (whether high flow or not) may be delivered to the patient via a nasal cannula. Delivering high flows of oxygen gas (for example, flows as high as 60 L/min) via a nasal cannula, may be more effective at rapidly pre-oxygenating the patient and can be used to effectively manage the volume of anatomical deadspace (e.g. the volume of gases in the airway of the patient that can potentially be re-breathed) such that rises in blood carbon dioxide concentration can be minimized.

Alternatively, pre-oxygenation may be delivered using other methods, including a facemask with an oxygen reservoir, non re-breather masks, self-inflating bag-valve-masks with or without one-way valves.

Delivering oxygen through high flow therapy e.g. flow rates above 10L/min can achieve pre-oxygenation to higher levels faster than traditional methods using a facemask with an oxygen reservoir, non re-breather masks, self-inflating bag-valve-masks with or without 1-way valves. Other high flow rates are contemplated, such high flow rates being those as described herein.

Flows delivered can be humidified to minimize potential tissue damage that might occur from the delivery of high flows of dry gas for a prolonged period of time. Referring to Figures 8 to 10, an alternative method will now be described. The alternative embodiment method and system have similar features and functions to the methods and systems described and described earlier and like numbers are used to indicate like parts.

Figure 8 shows an alternative system/apparatus 100 for determining dose/oxygenation requirements (hereinafter "oxygen requirements") of a patient for/in relation to anaesthesia (that is, the oxygen requirements pre-anaesthesia during a pre-oxygenation phase and/or the oxygen requirements during anaesthesia - which might include when the patient is apnoeic or when the patient is breathing). In an alternative embodiment the same method can be executed using the system disclosed in figure 1. The system/apparatus 100 is also configured to adjust parameters to provide high flow gas to a patient for the purposes of anaesthesia, and adjust the parameters of the high flow gas delivered to the patient as required to meet oxygenation requirements. The system/apparatus of Figure 8 is an alternative to the system of figure 1.

The system/apparatus of Figure 8 100 could be an integrated or separate component based arrangement, generally shown in the dotted box 151 in Figure 8. One or more sensors 158a, 158b, 158c, 158d, such as flow, oxygen, pressure, humidity, temperature or other sensors can be placed throughout the system and/or at, on or near the patient 156. The sensors can include a pulse oximeter 158d on the patient for determining the oxygen concentration in the blood.

In the embodiment of figure 8, the controller 108 is coupled to the flow source 102, humidifier 104 and sensors 158a-158d. Similar to the embodiment described in relation to figure 1, the controller 108 can operate the flow source 102 to provide the delivered flow of gas. It can control the flow, pressure, composition (where more than one gas is being provided), volume and/or other parameters of gas provided by the flow source 102 based on feedback from sensors (that is based on signals or other input received from the sensors indicating the parameter being sensed). The controller 108 can also control any other suitable parameters of the flow source 102 to meet oxygenation requirements. The controller 108 can also control the humidifier 104 based on feedback from the sensors 158a-158d. Using input from the sensors 158a-158d, the controller can determine oxygenation requirements and control parameters of the flow source 102 and/or humidifier 104 as required.

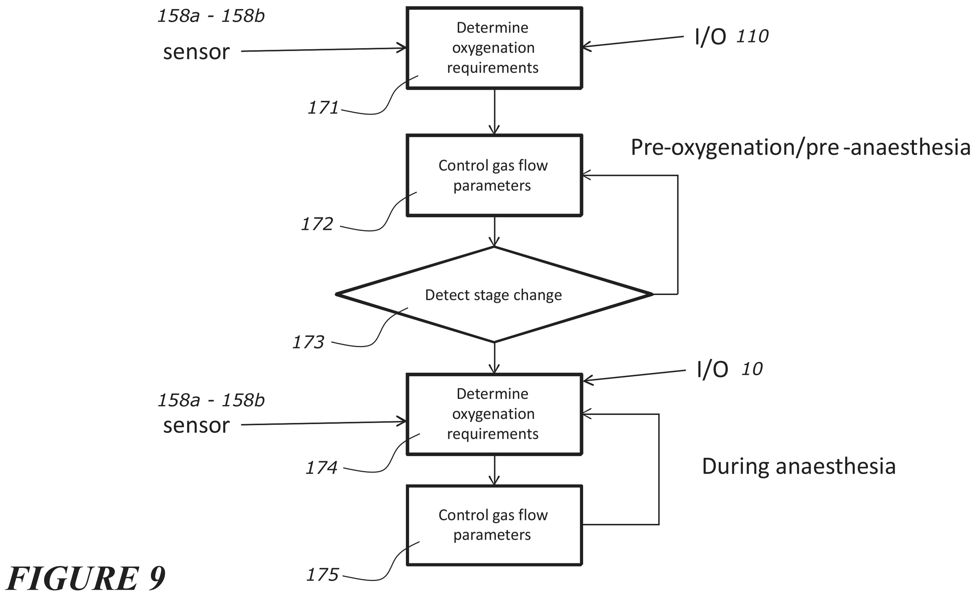

Referring to the flow diagram in Figure 9, the method using the system of Figure 8 will be described. The method of figure 9 may be implemented using the system of figure 1. The method of figure 9 may be executed in addition to or alternatively to steps 401 to 425 of figure 5.

The controller 108 is configured to carry out the determination of oxygen requirements and to control the parameters of high gas flow in accordance with that determination. First, during a pre-anaesthesia stage, the controller 108 determines oxygenation requirements of the patient, step 171. These can be oxygenation requirements that are based on the prediction of what might be required before and/or during anaesthesia based on historical/empirical data. The controller 108 receives input from the sensors 158a-158d and/or the user via the input interface 110. From that input and/or stored data (such as look up tables, historical data, parameters, relationships, the graphs or the like) the controller 108 determines the oxygenation requirement, step 171. The determination could take place through any processing, look up table, relationship (empirical or mathematical) or the like. Non-exhaustive examples of such input and determination processing are as follows. One or more alone or in combination could be used to make the oxygen requirement determination.

The user (such as anaesthetist or other clinician, or the patient) provides, input via the interface 110, a pre-operative assessment to estimate the level of risk for every patient. This level of risk relates to the risk of the patient entering hypoxia during anaesthesia. The controller 108 then determines oxygenation requirements, step 171, based on the level of risk and/or the user (e.g. anaesthetist or clinician) provides input indicative of the actual oxygenation requirement and/or dose/therapy settings and/or the actual parameter settings for the high flow gas delivery. Any of the input could be provided as a setting or range of settings or as one or more input values. The system could alert the user of the recommended settings or control the system to provide the settings, as to be described later.

Alternatively or additionally, and more generally, the user enters information from which oxygenation requirements can be determined, such information not necessarily directly indicating risk levels, or not being indicative of risk levels at all.

Sensor input could be used alternatively or additionally.

Next, once oxygenation requirements are determined, the controller 108 operates the flow source 102, humidifier 104 and/or other aspects of the system 100 to control the parameters of the high flow gas 153 delivered to the patient, step 172, so that the gas flow 153 meets the oxygenation requirements during a pre-anaesthesia (pre- oxygenation) stage. This can comprise altering one or more of:

- flow rate of gas (such as flow rate of oxygen)

- volume of gas delivered

- pressure of gas

- composition and/or concentration of gas

Examples of user input for determining oxygenation requirements and the resultant parameter settings are as follows.

The user enters the value on a scale. For example the user could choose a number from 1 (minimal risk) to 10 (high risk). The system could then choose the optimal settings for that scale number.

The user enters information such as age, weight, BMI, lung volumes, metabolic rate, body fat measure (e.g. percentage) and/or other patient factors that could be used individually or any combination to choose the optimal therapy settings (oxygen requirements). For example, a sum score method could be used with two or more of the factors listed. This can be used to predict the level of support (oxygenation) that will be required

The user enters pre-existing patient conditions. For example, if a patient is at risk of barotrauma the flow could be minimised to meet peak inspiratory demand but not deliver excess flow.

Existing limits on hardware could be used to choose the optimal therapy settings. For example, if the surgical environment is experiencing a shortage in oxygen the settings could be altered. 100% oxygen could be delivered only during inspiration and the flow could be set to meet the patient's peak inspiratory demand to ensure minimal wastage

Different levels of support could be optimal in different stages of undergoing anaesthesia. The high flow system 100 can optionally detect when a change in stage has occurred and alert the user or automatically determine new oxygenation requirements and/or change the gas flow parameters to me those new requirements. For example, after the pre-oxygenation stage, the patient is administered the anaesthesia and enters and anaesthesia stage. Breathing function can diminish and the patient can become apnoeic. Different oxygenation requirements exist to those pre-anaesthesia.

Therefore, the controller 108 is further configured to detect the anaesthesia stage (or change in anaesthesia stage), step 173. Possible methods for detecting a change in state are as follows.

The controller 108 uses the pressure waveform (from a pressure sensor) to detect when the patient is breathing or not (e.g. transition from pre-oxygenation to apnoea).

The controller 108 uses the expired $CO_2$ waveform (form a sensor) to detect when the patient is breathing or not (e.g. transition from pre-oxygenation to apnoea)

While the controller 108 is monitoring the state, step 162, the high flow gas 153 is delivered as per the parameters previously determined and set. After a change in stage is determined (such as transitioning from the pre-oxygenation stage to the anaesthesia stage) the controller/system 108/100 can continue delivering gas flow 153 with the same parameter settings. However, the system 100 can also go into a monitoring phase, step 174, wherein by the oxygenation requirements are re-determined, optionally in a continuously or periodic manner, step 174. Again previous or fresh input from a user via the input interface 110 can be used to determine the oxygenation requirements, in addition or alternatively to using sensor input 158a-158d. The oxygenation requirement can be determined in the same manner as described above for the pre-oxygenation stage, with the possible difference being that it is re-determined continuously or periodically based on updated input from the sensors and/or user.

The gas flow 153 parameters are then adjusted by the controller 108 to meet new oxygen requirements, these parameters being the same as described above, step 175. Even if updated input is not received, the oxygenation requirement might be re-determined on the basis that the stage of anaesthesia has changed, or alternatively the oxygenation requirement is not specifically re-determined, but a different oxygenation requirement is presumed and the high flow gas parameters are set accordingly for the new stage.

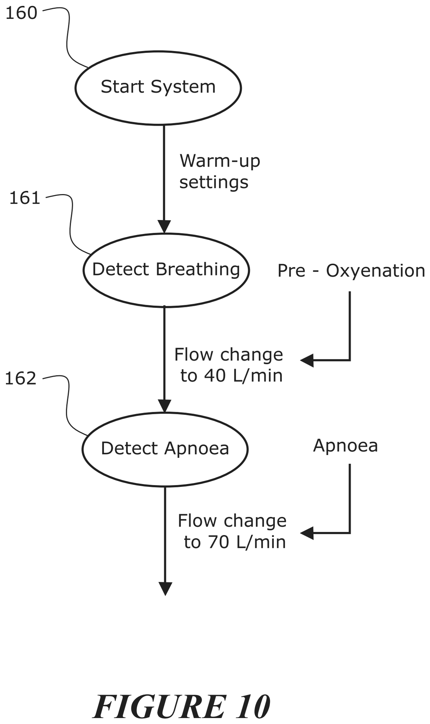

A particular non-limiting example of the function due to change in anaesthesia state is shown in Figure 10. After the system starts, step 160, the system monitors the patient and detects breathing, step 161, and determines a pre-oxygenation stage. The system provides gas flow parameters, including a flow rate of at least 40 L per minute, which are suitable for the pre-oxygenation stage, based on typical oxygenation requirements. After further monitoring of the patient, the system detects an apnoea, and assumes that the anaesthesia stage has started, step 162. That changes the parameters of the gas flow to a flow rate of at least 70 L per minute which meets the oxygenation requirements of the apnoeic stage, step 162. The controller may vary the flow rate based on any of the methods described to ensure the $SpO_2$ levels are maintained.

A continuous supply of oxygen and removal of carbon dioxide is essential to sustain healthy respiratory function. In addition to the method described above, the system can also be configured to monitor supply of oxygen and removal of carbon dioxide, step 174. Possible non-limiting methods of monitoring these comprise:

- monitoring expired $O_2$ and $CO_2$

- monitoring transcutaneous $O_2$ and $CO_2$

- monitoring blood gases (e.g. pulse oximeter)

- monitoring $SpO_2$.

$SpO_2$ may be monitored using any suitable sensor like a gas analyser or pulse oximeter.

In step 174, the trends/values of these parameters could be used to detect when the therapy settings (gas flow parameters) could be changed. The system is configured to then alert the user or automatically control the therapy dose (that is, gas flow parameters).

For example, if the $SpO_2$ starts to decrease past 90%, or other levels determined by the clinician as desirable from their assessment of the patient, for example, 91%, 92%, 93%, 94%, or 95%, the flow and or oxygen concentration (if not already at 100%) could increase to provide a higher level of support, step 175. If the end-tidal $CO_2$ value or trend shows an increase, the therapy support could increase as a higher level of support is needed, step 175. This should not be limited to oxygen and carbon dioxide. Other measured parameters (e.g. heart rate, blood pressure) could also be used to change the therapy dose settings.

During the preoxygenation stage, any suitable preoxygenation titration method may be used, such as the method of figure 5. During the apnoeic stage, any suitable preoxygenation titration method may be used, such as the method of figure 6.

In further embodiments, when the predicted or monitored pre-oxygenation or apnoeic time is small, the gas parameters can be changed accordingly. For example, if the estimated time of the anaesthesia stages (pre-oxygenation or during anaesthesia/apnea) is too short, the gas parameters can be adjusted to provide a higher level of support for more time - for example the oxygen concentration, flow rate, oxygen volume, pressure and/or gas composition can be changed, for example. Various methods of predicting the duration of the stages could be used.

As relatively high gas delivery flow rates may be used with the embodiments or configurations described herein, the gases being supplied or delivered to the user or patient can may be delivered to different parts of the user's or a patient's airway.

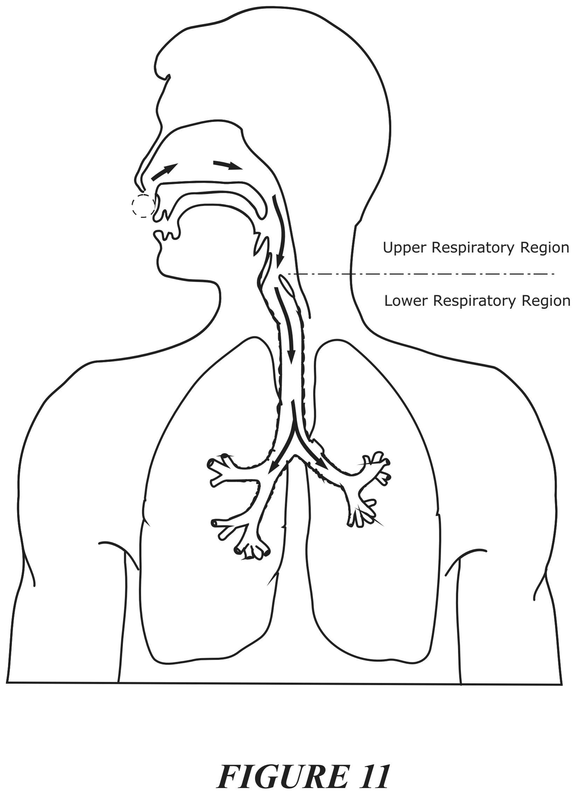

Such relatively high flow rates of gases may assist in providing the supplied gases into a user's airway, or to different parts of a user's airway, for example such flow rates may allow for a delivery of such gases to the upper or lower airway regions as shown in Figure 11. Upper airway region typically includes the nasal cavity, pharynx and larynx, while the lower airway region typically includes the trachea, primary bronchi and lungs.

The embodiments described can utilise the knowledge of the respiratory flow wave and/or the transition between inspiration and expiration. Possible methods and apparatus for respiratory flow wave, meeting (e.g. peak) inspiratory demand and estimating (e.g. peak) inspiratory demand can be used. It should also be noted that the following can utilise switching modes of operation between inspiration and expiration. The exact moment of switching should not be limited to the exact transition point.

In some configurations the information related to the patient can be accessed remotely from a database. Further the system can automatically provide therapy settings based on stored patient information. Also a clinician can remotely operate the system.

**EP 4 740 987 A2**

The foregoing description of the invention includes preferred forms thereof. Modifications may be made thereto without departing from the scope of the invention.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to."

Where, in the foregoing description reference has been made to integers or components having known equivalents thereof, those integers or components are herein incorporated as if individually set forth.

The disclosed methods, apparatus and systems may also be said broadly to comprise the parts, elements and features referred to or indicated in the disclosure, individually or collectively, in any or all combinations of two or more of said parts, elements or features.

Recitation of ranges herein is merely intended to serve as a shorthand method of referring individually to each separate sub-range or value falling within the range, unless otherwise indicated herein, and each separate sub-range or value is incorporated into the specification as if it were individually recited herein.

Reference to any prior art in this specification is not, and should not be taken as, an acknowledgement or any form of suggestion that that prior art forms part of the common general knowledge in the field of endeavour in any country in the world.

Certain features, aspects and advantages of some configurations of the present disclosure have been described with reference to use of the gas humidification system with a respiratory therapy system. However, certain features, aspects and advantages of the use of the gas humidification system as described may be advantageously be used with other therapeutic or non-therapeutic systems requiring the humidification of gases. Certain features, aspects and advantages of the methods and apparatus of the present disclosure may be equally applied to usage with other systems.

Although the present disclosure has been described in terms of certain embodiments, other embodiments apparent to those of ordinary skill in the art also are within the scope of this disclosure. Thus, various changes and modifications may be made without departing from the spirit and scope of the disclosure. For instance, various components may be repositioned as desired. Moreover, not all of the features, aspects and advantages are necessarily required to practice the present disclosure. Accordingly, the scope of the present disclosure is intended to be defined only by the claims that follow.

**ITEMS**

A1. A respiratory therapy system comprising:

a flow source or flow generator adapted to provide a gas flow to a patient,

a sensor adapted to measure a first quantity of nitrogen inhaled by the patient $N_i$ and a second quantity of nitrogen exhaled by the patient $N_o$, and a hardware controller configured to:

control the flow source or flow generator to deliver a first flow therapy,

receive the measured $N_i$ and $N_o$ over at least one respiratory cycle, and

control the flow source or flow generator to continue delivering the first flow therapy or to deliver a second flow therapy on the basis of a function of the measured $N_i$ and $N_o$.

A2. The respiratory therapy system of item A1, wherein if the $N_o$ is less than or equal to the $N_i$ plus a predetermined nitrogen variance value $N_p$, the flow source or flow generator is controlled to deliver the second flow therapy.

A3. The respiratory therapy system of item A1, wherein if the ratio of $N_o$:$N_i$ is less than or equal to a predetermined nitrogen ratio $N_{p\_r}$, the flow source or flow generator is controlled to deliver the second flow therapy.

A4. The respiratory therapy system of item A1, wherein different concentrations of gases are delivered in the second flow therapy than in the first flow therapy.

A5. The respiratory therapy system of item A1, wherein the first flow therapy comprises delivery of a first gas composition comprising a first level of ambient gas $G_{a1}$ and a first level of oxygen $G_{o1}$, and wherein the second flow therapy comprises delivery of a second level of ambient gas $G_{a2}$ and a second level of oxygen $G_{o2}$.

28

A6. The respiratory therapy system of item A6, wherein $G_{o1} > G_{o2}$.

A7. The respiratory therapy system of item A6, wherein $G_{o1}:(G_{a1+}G_{o1}) > G_{o2}:(G_{a2+}G_{o2})$.

A8. A respiratory therapy kit comprising:

a patient interface,

a humidifier comprising an inlet adapted to be connected to a flow source or flow generator, and

a conduit adapted to allow for fluid communication between the humidifier and the patient interface,

wherein the patient interface, the conduit, and at least a part of the humidifier are integrally moulded or permanently connected.

A9. The respiratory therapy kit of item A8, wherein the humidifier further comprises a fluid reservoir.

A10. The respiratory therapy kit of item A9, further comprising a heating device comprising a chemical heater adapted to heat fluid in the fluid reservoir.

A11. The respiratory therapy kit of item A10, wherein the heating device comprises a manually actuatable switch configured to activate the chemical heater.

A12. The respiratory therapy kit of item A8, wherein the patient interface comprises a port configured to accept a drug delivery device.

A13. The respiratory therapy kit of item A12, further comprising a drug delivery device adapted to be located in the port, wherein the drug delivery device comprises a manually actuatable metering mechanism configured to allow for the release of medication.

A14. A respiratory therapy system, comprising:

a respiratory therapy apparatus,

a patient interface configured to be removably attachable to a conduit, and

a conduit adapted to extend between the respiratory therapy apparatus and the patient interface,

wherein the respiratory therapy apparatus and the conduit are integrally moulded or permanently connected.

A15. The respiratory therapy system of item A15, further comprising a disinfection arrangement adapted to disinfect at least a section of the conduit.

A16. The respiratory therapy system of item A16, further comprising a hardware controller configured to control the disinfection arrangement, wherein the hardware controller controls the disinfection arrangement dependent on whether or not the patient interface is attached to the conduit.

A17. The respiratory therapy system of item A16, further comprising a hardware controller configured to control the disinfection arrangement, wherein the hardware controller is configured to activate the disinfection arrangement after removal of the patient interface from the conduit.

A18. The respiratory therapy system of items A17 or A18, further comprising a flow sensor or pressure sensor, wherein the hardware controller is configured to, in use, utilize signals from the flow sensor or pressure sensor or values derived therefrom to determine if the patient interface is or is not attached to the conduit.

A19. The respiratory therapy system of item A15, further comprising a one-way valve adapted to prevent the flow of exhaled gases into the conduit.

A20. The respiratory therapy system of item A15, wherein the respiratory therapy apparatus comprises a flow source or flow generator, a humidifier, or an integrated flow source or flow generator/humidifier apparatus.

B1. A method for estimating and/or determining one or more pre-oxygenation parameters for a patient undergoing pre-oxygenation therapy, comprising one or more of:

measuring and/or tracking the concentration of one or more respiratory gases in the patient's expired gas,

measuring and/or tracking the concentration of one or more respiratory gases in the patient's blood (blood saturation),

measuring and/or tracking the concentration of a tracer introduced to the patient's respiratory tract,

measuring and/or tracking one or more physical properties of the patient's expired gas.

B2. The method of item B1, wherein the one or more pre-oxygenation parameters comprise(s):

the time until a patient undergoing a pre-oxygenation procedure has been sufficiently pre-oxygenated,

the likelihood of a pre-oxygenation procedure achieving a target end-tidal oxygen level for a patient,

the end-tidal oxygen level for a patient undergoing a pre-oxygenation procedure,

if a patient has been sufficiently pre-oxygenated during a pre-oxygenation procedure,

changes in pre-oxygenation level of a patient undergoing a pre-oxygenation procedure.

B3. The method of item B1 or B2, wherein said respiratory gas is one or more of oxygen and nitrogen.

B4. The method of any one of the preceding B items, wherein the step of measuring and/or tracking the concentration of one or more respiratory gases in the patient's expired gas is performed using a mass spectrometer.

B5. The method of any one of the preceding B items, wherein the step of measuring and/or tracking the concentration of one or more respiratory gases in the patient's expired gas is performed using a gas analyser.

B6. The method of item B4, wherein said gas analyser is one or more of an oxygen gas analyser and a nitrogen gas analyser.

B7. The method of any one of the preceding B items, wherein the step of measuring and/or tracking the concentration of one or more respiratory gases in the patient's blood is performed via pulse oximetry.

B8. The method of any one of the preceding B items, wherein the tracer is one or more of nitrogen, helium, argon and sulfur-hexafluoride.

B9. The method of any one of the preceding B items, wherein the concentration of the tracer is measured and/or tracked using one or more of:

an emission spectrometer

an ultrasonic gas analyser

a mass spectrometer.

B10. The method of any one of the preceding B items, wherein the tracer is introduced to the patient's respiratory tract via inhalation prior to delivery of the pre-oxygenation therapy.

B11. The method of any one of items B1 to B10, wherein the tracer is introduced to the patient's respiratory tract via inhalation during an initial warm-up phase of the pre-oxygenation therapy.

B12. The method of any one of the preceding B items, wherein the step of measuring and/or tracking the one or more physical properties of the patient's expired gas is used to estimate the fractional concentrations of one or more respiratory gases in the expired gas.

B13. The method of any one of the preceding B items, wherein said one or more physical properties comprises:

density,

viscosity,

acoustic response.

B14. The method of any one of the preceding B items, wherein said one or more measuring and/or tracking steps is performed continuously.

B15. The method of item B14, wherein said continuous measurement and/or tracking is performed until the patient is sufficiently pre-oxygenated.

B16. The method of any one of the preceding B items, wherein sufficient pre-oxygenation is indicated by a sustained end-tidal oxygen concentration and/or blood oxygen saturation level greater than 90%.

B17. The method of any one of the preceding B items, wherein sufficient pre-oxygenation is indicated by substantially steady-state concentration(s) and/or blood saturation level(s) of said one or more respiratory gases and/or said tracer.

B18. The method of any one of the preceding B items, further comprising the steps of:

measuring and/or estimating the rate of change of the patient's expired concentration and/or blood saturation of said one or more respiratory gases and/or tracer

fitting the rate of change to a non-linear model

determining a time constant for said model

multiplying the time constant with a treatment factor to estimate the time until sufficient pre-oxygenation may be achieved.

B19. The method of item B18, wherein the non-linear model is an exponential curve.

B20. The method of item B19, wherein the treatment factor is four.

B21. The method of any one of items B18 to B20, wherein said time until sufficient pre-oxygenation is further multiplied by a safety factor.

B22. The method of any one of items B18 to B21, wherein the time constant is the time taken for the concentration of nitrogen in the expired gas to drop to substantially 37% of the concentration of nitrogen in the first measurement of expired gas.

B23. The method of any one of items B18 to B21, wherein the time constant is the time taken for the concentration of tracer in the expired gas to drop to substantially 37% of the concentration of tracer in the first measurement of expired gas.

B24. The method of any one of items B18 to B21, wherein the time constant is the time taken for the concentration of oxygen in the expired gas to increase by substantially 63% of the concentration of oxygen in the first measurement of expired gas.

B25. The method of any one of items B18 to B21, wherein the time constant is the time taken for the blood saturation of oxygen to increase by substantially 63% of the patient's blood saturation of oxygen prior to or at the start of the pre-oxygenation procedure.

B26. The method of any one of items B18 to B21, wherein the time constant is the quotient of dividing the patient's functional residual capacity by the patient's alveolar ventilation.

B27. The method of any one of the preceding B items, wherein pre-oxygenation is delivered via high flow therapy.

B28. The method of any one of the preceding B items, wherein pre-oxygenation is delivered via a nasal cannula.

B29. Apparatus configured for estimating and/or determining one or more pre-oxygenation parameters for a patient undergoing pre-oxygenation therapy comprising one or more sensors configured to:

measure and/or track the concentration of one or more respiratory gases in the patient's expired gas and/or

measure and/or track the concentration of one or more respiratory gases in the patient's blood (blood saturation) and/or

measure and/or track the concentration of a tracer introduced to the patient's respiratory tract and/or

measure and/or track one or more physical properties of the patient's expired gas.

B30. The apparatus of item B29, wherein said one or more pre-oxygenation parameters comprise(s):

the time until a patient undergoing a pre-oxygenation procedure has been sufficiently pre-oxygenated,

the likelihood of a pre-oxygenation procedure achieving a target end-tidal oxygen level for a patient,

the end-tidal oxygen level for a patient undergoing a pre-oxygenation procedure,

if a patient has been sufficiently pre-oxygenated during a pre-oxygenation procedure,

changes in pre-oxygenation level of a patient undergoing a pre-oxygenation procedure.

B31. The apparatus of item B29 or B30, further comprising a processor configured to:

fit the rate of change of the patient's expired concentration and/or blood saturation of said one or more respiratory gases and/or tracer to a non-linear model,

determine a time constant for said model,

multiply the time constant with a treatment factor to estimate the time until sufficient pre-oxygenation may be achieved,

indicate when the patient has been sufficiently pre-oxygenated.

B32. The apparatus of item B31, further comprising a timer configured to indicate the estimated time remaining until the patient is sufficiently pre-oxygenated.

B33. The apparatus of any one of items B29 to B32 further comprising a control interface or a connection to a control interface of a pre-oxygenation therapy apparatus or system providing pre-oxygenation therapy to said patient.

B34. A system for estimating and/or determining one or more pre-oxygenation parameters for a patient undergoing pre-oxygenation therapy, the system configured to implement the method of any one of items B1 to B28.

B35. A system for estimating and/or determining one or more pre-oxygenation parameters for a patient undergoing pre-oxygenation therapy, the system comprising a patient interface and the apparatus of any one of items B29 to B33.

**10402**

C 1. A method of oxygenating a patient in relation to anaesthesia using high flow gas delivery comprising determining oxygenation requirements of the patient before or during anaesthesia.

C 2. A method according to item C1 wherein determining oxygenation requirements comprises one or more of:

receiving input indicative of risk assessment

receiving input indicative of oxygenation requirements of the patient;

receiving input relating to patient physiology and using the input to determine oxygenation requirements of the patient, optionally wherein the input could be one or more of patient:

age,

weight,

height,

body fat measure (e.g. percentage)

BMI,

lung volume,

metabolic rate;

receiving input relating to pre-existing patient conditions and using the input to determine oxygenation requirements of the patient;

sensing physiological parameters of the patient and using that to determine oxygenation requirements of the patient;

monitoring O2 supply to and/or CO2 removal from the patient and from that determining oxygenation requirements;

ascertaining limits on apparatus delivering the high flow gas and using that to determine oxygenation requirements;

determining the stage of anaesthesia and using that to determine oxygenation requirements;

determining the expected or monitoring the actual duration of one or more stages of anaesthesia and using that to determine oxygenation requirements;

receiving input of actual high flow gas parameter settings required for the oxygenation requirement.

C 3. A method according to item C2 wherein the stages of anaesthesia comprise one or more of:

pre-anaesthesia where the patient is breathing (pre-oxygenation),

during anaesthesia where the patient is apnoeic.

C 4. A method according to item C2 or C3 further comprising detecting the stage of anaesthesia by detecting breathing pressure to determine if the patient is: a) breathing and in the pre-oxygenation state, or b) not breathing and in the apnoeic stage.

C 5. A method according to item C2, C3 or C4 further comprising detecting the stage of anaesthesia by detecting the expired CO2 to determine if the patient is: a) breathing and in the pre-oxygenation state, or b) not breathing and in the apnoeic stage.

C 6. A method according any one of items C2 to C5 wherein monitoring O2 supply and/or CO2 removal comprises monitoring one or more of:

expired O2, CO2,

transcutaneous O2, CO2,

blood gases,

SpO2.

C 7. A method according to any one of the preceding C items further comprising controlling one or parameters of the high flow gas to assist oxygenation of the patient according to the oxygenation requirements.

C 8. A method according to item C7 wherein controlling one or more parameters of the high flow gas comprises controlling one or more of:

flow rate of gas (such as flow rate of oxygen)

volume of gas delivered

pressure of gas

composition and/or concentration of gas.

C 9. A method according to item C7 or C8 further comprising, upon monitoring O2 supply and/or CO2 removal, increasing flow and/or oxygen concentration if:

SpO2 decreases past 90%,

End tidal CO2 increases.

C 10. A method according to item C7, C8 or C9 wherein, upon determining a short duration of one or more stages of anaesthesia, increasing the flow and/or oxygen concentration.

C 11. A method according to any preceding item C, wherein the high flow gas is delivered and the oxygenation requirements are determined using a high flow therapy apparatus.

C 12. A system for oxygenating a patient in relation to anaesthesia using high flow gas delivery comprising:

a flow source, and

a controller for determining oxygenation requirements of the patient before or during anaesthesia.

C 13. A system according to item C12 wherein determining oxygenation requirements comprises one or more of:

receiving input indicative of risk assessment

receiving input indicative of oxygenation requirements of the patient;

receiving input relating to patient physiology and using the input to determine oxygenation requirements of the patient, optionally wherein the input could be one or more of patient:

age,

weight,

height,

body fat measure (e.g. percentage)

BMI,

lung volume,

metabolic rate;

receiving input relating to pre-existing patient conditions and using the input to determine oxygenation requirements of the patient;

sensing physiological parameters of the patient and using that to determine oxygenation requirements of the patient;

monitoring O2 supply to and/or CO2 removal from the patient and from that determining oxygenation requirements;

ascertaining limits on apparatus delivering the high flow gas and using that to determine oxygenation requirements;

determining the stage of anaesthesia and using that to determine oxygenation requirements;

determining the expected or monitoring the actual duration of one or more stages of anaesthesia and using that to determine oxygenation requirements;

receiving input of actual high flow gas parameter settings required for the oxygenation requirement.

C 14. A system according to item C13 wherein the stages of anaesthesia comprise one or more of:

pre-anaesthesia where the patient is breathing (pre-oxygenation),

during anaesthesia where the patient is apnoeic.

C 15. A system according to item C13 or C14 wherein the controller further detects the stage of anaesthesia by detecting breathing pressure to determine if the patient is: a) breathing and in the pre-oxygenation state, or b) not breathing and in the apnoeic stage.

C 16. A system according to item C13, C14 or C15 wherein the controller further detects the stage of anaesthesia by detecting the expired CO2 to determine if the patient is: a) breathing and in the pre-oxygenation state, or b) not breathing and in the apnoeic stage.

C 17. A system according any one of items C13 to C16 wherein monitoring O2 supply and/or CO2 removal comprises monitoring one or more of:

expired O2, CO2,

transcutaneous O2, CO2,

blood gases,

SpO2.

C 18. A system according to any one of items C13 to C17 wherein the controller further controls one or parameters of the high flow gas to assist oxygenation of the patient according to the oxygenation requirements.

C 19.A system according to item C18 wherein controlling one or more parameters of the high flow gas comprises controlling one or more of:

flow rate of gas (such as flow rate of oxygen)

volume of gas delivered

pressure of gas

composition and/or concentration of gas.

C20. A system according to item C18 or C19 further comprising the controller, upon monitoring O2 supply and/or CO2 removal, increasing flow and/or oxygen concentration if:

SpO2 decreases past 90%,

End tidal CO2 increases.

C21. A system according to item C18, C19 or C20 wherein, upon determining a short duration of one or more stages of anaesthesia, the controller increases the flow and/or oxygen concentration.

**REPRESENTATIVE FEATURES**

**1.** A system for oxygenating a patient in relation to anaesthesia using high flow gas delivery comprising:

a flow source, and

a controller for determining oxygenation requirements of the patient before or during anaesthesia.

**2.** The system according to clause 1, wherein the controller is adapted to control the flow and/or oxygen concentration of the high flow gas to assist oxygenation of the patient according to the oxygenation requirements.

**3.** The system according to clause 2, wherein the controller is adapted to maintain the flow and/or oxygen concentration of the high flow gas.

**4.** The system according to any clause 1 or 2, wherein the controller is adapted to increase flow and/or oxygen concentration of the high flow gas to assist oxygenation of the patient if sufficient oxygenation has not occurred.

**5.** The system according to any one of the preceding clauses, wherein the controller includes a timer adapted to indicate the period of time over which gases have been delivered.

**6.** The system according to clause 4, wherein the controller is adapted to determine if sufficient oxygenation has not occurred by monitoring one or more respiratory gases and/or monitoring the patient's blood oxygen saturation level.

**7.** The system according to clause 6, wherein the one or more respiratory gases comprises oxygen and the system comprises an oxygen gas analyser.

**8.** The system according to clause 7, wherein the controller is adapted receive input indicating a first quantity of oxygen inhaled by the patient Oi and a second quantity of oxygen exhaled by the patient Oo, and the controller is configured to:

control the flow source to deliver a first flow therapy,
receive the input indicating Oi and Oo over at least one respiratory cycle, and
control the flow source to continue delivering the first flow therapy or to deliver a second flow therapy on the basis of a function of the Oi and Oo.

**9.** The system according to any one of clauses 6 to 8, wherein the one or more respiratory gases comprises nitrogen and the system comprises a nitrogen gas analyser.

**10.** The system according to clause 9, wherein the controller is adapted to receive input indicating a first quantity of nitrogen inhaled by the patient Ni and a second quantity of nitrogen exhaled by the patient No, and the controller is configured to:

control the flow source to deliver a first flow therapy,
receive input indicating Ni and No over at least one respiratory cycle, and
control the flow source to continue delivering the first flow therapy or to deliver a second flow therapy on the basis of a function of the Ni and No.

**11.** A method of oxygenating a patient in relation to anaesthesia using high flow gas delivery comprising determining oxygenation requirements of the patient before or during anaesthesia.

**12.** The method according to clause 11, further comprising controlling the flow and/or oxygen concentration of the high flow gas to assist oxygenation of the patient according to the oxygenation requirements.

**13.** The method according to clause 12, further comprising maintaining the flow and/or oxygen concentration of the high flow gas.

**14.** The method according to any clause 11 or 12, further comprising increasing the flow and/or oxygen concentration of the high flow gas to assist oxygenation of the patient if sufficient oxygenation has not occurred.

**15.** The method according to clause 14, wherein determining if sufficient oxygenation has not occurred comprises monitoring one or more respiratory gases and/or monitoring the patient's blood oxygen saturation level.

**16.** The method according to any one of clauses 11 to 15, further comprising timing the period of time over which gases have been delivered.

**17.** The method according to clause 15 or clause 16, wherein monitoring the one or more respiratory gases comprises monitoring oxygen.

**18.** The method according to clause 17, wherein monitoring oxygen comprises receiving input indicating a first quantity of oxygen inhaled by the patient Oi and a second quantity of oxygen exhaled by the patient Oo, and:

controlling the flow source to deliver a first flow therapy,

receiving the input indicating Oi and Oo over at least one respiratory cycle, and

controlling the flow source to continue delivering the first flow therapy or to deliver a second flow therapy on the basis of a function of the Oi and Oo.

**19.** The method according to any one of clauses 15 to 18, wherein the one or more respiratory gases comprises nitrogen and the method comprising monitoring nitrogen.

**20.** The method according to clause 19, wherein monitoring nitrogen comprises receiving input indicating a first quantity of nitrogen inhaled by the patient Ni and a second quantity of nitrogen exhaled by the patient No, and: controlling the flow source to deliver a first flow therapy, receiving the input indicating Ni and No over at least one respiratory cycle, and controlling the flow source to continue delivering the first flow therapy or to deliver a second flow therapy on the basis of a function of the Ni and No.

**21.** A system for oxygenating a patient in relation to anaesthesia using high flow gas delivery comprising:

a flow source, and

a controller for determining oxygenation requirements of the patient before anaesthesia when the patient is breathing.

**22.** The system according to clause 21 further wherein the controller is adapted to control the flow source to provide a high flow gas flow.

**23.** The system according to clause 22, wherein the controller is adapted to control the flow source to provide an initial gas flow rate based on at least the BMI or any other patient parameters in combination with BMI.

**24.** The system according to clause 23, wherein the initial gas flow rate is above 30 L/min.

**25.** The system according to clause 22, wherein, upon monitoring the transcutaneous O2 level, the controller is adapted to increase oxygen concentration in the gas flow if the oxygen saturation level is lower than 99%, the transcutaneous O2 level is lower than 380mmHg, and the transcutaneous O2 level minus a predetermined value is lower than a previous value.

**26.** The system according to clause 22, wherein, upon monitoring the transcutaneous CO2 level, the controller is adapted to increase oxygen concentration of the gas flow if the transcutaneous CO2 level is greater than 30mmHg and if the new transcutaneous CO2 level is greater than a previous saturation level plus a predetermined value.

**27.** The system according to clause 22, wherein, upon monitoring blood oxygen saturation level, the controller is adapted to produce a warning if the blood oxygen saturation level has fallen.

**28.** The system according to clause 22, wherein, upon monitoring blood oxygen saturation and transcutaneous CO2, the controller is adapted to indicate the end of the pre-oxygenation phase if the oxygen saturation is greater than 99% and the transcutaneous CO2 is equal to or less than 30mmHg.

**29.** A system for oxygenating a patient in relation to anaesthesia using high flow gas delivery comprising:

a flow source, and

a controller for determining oxygenation requirements of the patient during anaesthesia when the patient is apnoeic.

**30.** A system according to clause 29 further wherein the controller is adapted to control the flow source to provide a high flow gas flow.

**31.** The system according to clause 30, wherein, upon monitoring blood oxygen saturation level, the controller is adapted to produce a warning if the blood oxygen saturation level is less than 92%.

**32.** The system according to clause 31, wherein the controller is adapted to control the flow source to provide an initial gas flow rate based on at least the BMI or any other patient parameters in combination with BMI such that flow rate is proportional to BMI.

**33.** The system according to clause 32, wherein the initial gas flow rate is above 70 L/min.

**34.** The system according to clause 30, wherein upon monitoring the blood oxygen saturation, the controller is adapted to increase the flow of the gas flow if the average rate of change of blood oxygen saturation is negative, and if the rate of change of blood oxygen saturation is not increasing, and if the flow or 100L/minute or greater.

**35.** The system according to clause 30, wherein upon monitoring the blood oxygen saturation, the controller is adapted to increase the flow of the gas flow if the average rate of change of blood oxygen saturation is negative, if the flow is less than 100L/minute and if the rate of change of blood oxygen saturation is not increasing.

**36.** The system according to clause 30, wherein upon monitoring the blood oxygen saturation, the controller is adapted to warn the clinician if the average rate of change of blood oxygen saturation is negative, if the flow is 100L/minute or greater and if the oxygen concentration is 99% or greater.

**37.** The system according to clause 30, wherein upon monitoring the blood oxygen saturation, the controller is adapted to increase the flow and/or oxygen concentration of the gas flow if the average rate of change of blood oxygen saturation is negative and if the rate of change of blood oxygen saturation is increasing.

**38.** The system according to clause 30, upon monitoring the blood oxygen saturation, the controller is adapted to decrease oxygen concentration of the gas flow if the average rate of change of blood oxygen saturation is zero or positive and if the average level of blood oxygen saturation is 99% or greater.

This divisional application is divided from EP16752716.7 (the 'parent application') and the divisional specification as filed comprises the content of the parent application, including the original claims recited as 'representative features' above. The scope of this disclosure therefore includes the full content of the parent application. In any case, protection may be sought for any features disclosed in the parent application as filed.

**Claims**

1. An apparatus for estimating and/or determining one or more pre-oxygenation parameters for a patient undergoing pre-oxygenation therapy, the apparatus comprising:

   an unsealed nasal interface;
   a flow source configured to providing pre-oxygenation therapy to the patient via the unsealed nasal interface, wherein the flow source provides a flow of gases with a flow rate greater than 15 L/min;
   one or more sensors configured to measure and/or track the concentration of oxygen in the patient's expired gas;
   wherein the apparatus estimates and/or determines the one or more pre-oxygenation parameters from the sensor measurements.

2. The apparatus according to claim 1, wherein the one or more pre-oxygenation parameters comprise:

   a) the time until a patient undergoing a pre-oxygenation procedure has been sufficiently pre-oxygenated,
   b) the likelihood of a pre-oxygenation procedure achieving a target end-tidal oxygen level for a patient,
   c) the end-tidal oxygen level for a patient undergoing a pre-oxygenation procedure,
   d) if a patient has been sufficiently pre-oxygenated during a pre-oxygenation procedure,
   e) changes in pre-oxygenation level of a patient undergoing a pre-oxygenation procedure.

3. The apparatus according to claim 1 or claim 2, wherein measurements at or around the initial phase of the pre-oxygenation procedure may be used to predict the time remaining until there is sufficient pre-oxygenation.

4. The apparatus according to any one of claims 1 to 3, wherein the expired oxygen concentration is monitored throughout the pre-oxygenation process and the predicted time is adjusted as the process continues.

5. The apparatus according to any one of claims 1 to 4, wherein sufficient pre-oxygenation is indicated by one or both of:

   a sustained end-tidal oxygen concentration, optionally wherein the sustained end-tidal oxygen concentration is greater than 70%, such as 87% or greater than 90%; and
   blood oxygen saturation level greater than 90%.

6. The apparatus according to any one of claims 1 to 5, wherein the measuring and/or tracking of the concentration of oxygen in the patient's expired gas is performed using a mass spectrometer.

7. The apparatus according to any one of claims 1 to 5, wherein the measuring and/or tracking of the concentration of oxygen in the patient's expired gas is performed using a gas analyser, and optionally wherein the gas analyser is positioned proximal to the flow source.

8. The apparatus according to any one of claims 1 to 7, wherein the one or more sensors are configured to measure and/or track the concentration of oxygen in the patient's expired gas continuously.

9. The apparatus according to any one of claims 1 to 8, wherein the apparatus further comprises a processor configured to:

   a) estimate the rate of change of the patient's expired oxygen concentration; and/or
   b) fit a rate of change of the patient's expired oxygen concentration to a non-linear model, and/or
   b) determine a time constant for said model, and optionally

c) multiply the time constant with a treatment factor to estimate the time until sufficient pre-oxygenation may be achieved, and/or

d) indicate when the patient has been sufficiently pre-oxygenated.

wherein the time constant is the time taken for the concentration of oxygen in the expired gas to increase by substantially 63% of the difference between a desired level and the concentration of oxygen in a first measurement of expired gas.

10. The apparatus according to claim 9, wherein the processor further comprises a countdown timer configured to indicate estimated time remaining until the patient is sufficiently pre-oxygenated; and optionally wherein:

the timer has a user interface in the form of coloured lights; and/or
there is audio or visual indication when pre-oxygenation is complete.

11. The apparatus according to any one of claims 1 to 10, wherein the flow rate of gases provided is greater than or equal to about 20 L/min, or wherein the flow rate of gases provided is in a range from 30 L/min to 150 L/min.

12. The apparatus according to any one of claims 1 to 11, wherein the apparatus further comprises a humidifier to humidify the flow of gases.

13. The apparatus according to any one of claims 1 to 12, further comprising one or both of:

one or more sensors configured to measure and/or track the concentration of one or more respiratory gases in the patient's blood; and
wherein the measuring and/or tracking of the concentration of one or more respiratory gases in the patient's blood is performed via pulse oximetry.

14. The apparatus according to any one of claims 1 to 13, wherein the apparatus is further configured to display the concentration of oxygen in the patient's expired gas to a user.

15. The apparatus according to any one of claims 1 to 14, wherein the unsealed nasal interface comprises a sensing module comprising the one or more sensors configured to measure and/or track the concentration of oxygen in the patient's expired gas; and optionally wherein the sensing module comprises the gas analyser.

**FIGURE 1**

EP 4 740 987 A2

FIGURE 2

**FIGURE 3A**

**FIGURE 3B**

EP 4 740 987 A2

43

*FIGURE 4*

Phase 1 - Preoxygenation

**FIGURE 5**

Phase 2 - Patient is apnoeic

*473* Set 70l/min Flow @ 95% O2

*475* BMI > 27? —No→

*477* Set 90l/min Flow @ 60% O2

*479* BMI > 35? —No→

*481* Set 100l/min Flow @ 60% O2

*483* Begin Automatic Titration for Phase 2

Make 2 measurements of 02Sats, TcCo2 at 10 sec intervals

*487* Wait 10 sec

*485* Wait 10 sec

*489* Measure O2Sats, TcCO2, TcO2

*491* Calculate average rate of change of values over last 3 readings: Rav

*493* Calculate rate of change of values since last reading: Rinst

*495* O2Sats92%? —Yes→

*497* Warn Clinician of low O2 Sats

*499* Rav O2sats < 0? —Yes→

*501* Rinst O2 sats < Rav O2Sats? —Yes→

*503* Set 100l/min Flow @ 99% O2

*505* O2Sats >=99%?

*507* Decrease O2 Concentration by 1%

*509* Flow >= 100l/m? —No→

*511* Increase Flow by 5l/m

*513* O2 Concentration < 99%? —No→

*515* Warn Clinician that O2 and Flow are max

*517* Increase O2 Concentration by 1%

*519* Clinician disengages automatic titration

*521* Phase 2 auto titration loop is halted

*523* Physiological measurements made and displayed

*525* Wait 1 sec

*527* Autotitration reengaged? —No→ / Yes

FIGURE 6

46

*FIGURE 7*

EP 4 740 987 A2

EP 4 740 987 A2

**FIGURE 8**

*158a - 158b*

sensor → **Determine oxygenation requirements** ← I/O *110*

*171*

**Control gas flow parameters**

Pre-oxygenation/pre-anaesthesia

*172*

**Detect stage change**

*173*

*158a - 158b*

sensor → **Determine oxygenation requirements** ← I/O *10*

*174*

During anaesthesia

**Control gas flow parameters**

*175*

*FIGURE 9*

160 — ( Start System )

Warm-up
settings

161 — ( Detect Breathing )   Pre - Oxyenation

Flow change
to 40 L/min

162 — ( Detect Apnoea )   Apnoea

Flow change
to 70 L/min

# *FIGURE 10*

**FIGURE 11**

Upper Respiratory Region

Lower Respiratory Region

*FIGURE 12A*

*FIGURE 12B*

*FIGURE 12C*

**EP 4 740 987 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 16752716